# EUROPEAN PATENT SPECIFICATION

(11) **EP 2 554 552 B1**
(45) Date of publication and mention of the grant of the patent: **13.05.2015**
(21) Application number: 11759608.0
(22) Date of filing: 25.03.2011
(51) Int. Cl.: C07K 16/28, A61K 39/395, A61P 3/10, A61P 37/06, C12N 1/15, C12N 1/19, C12N 1/21, C12N 5/10, C12N 15/09, C12P 21/08

(54) **NOVEL ANTI-CD98 ANTIBODY AND USE THEREOF**
NEUER ANTIKÖRPER GEGEN CD98 UND VERWENDUNG DAVON
NOUVEL ANTICORPS ANTI-CD98 ET SON UTILISATION

(30) Priority: 08.12.2010 JP 2010273455; 08.09.2010 JP 2010200599; 26.03.2010 JP 2010072966
(43) Date of publication of application: 06.02.2013
(73) Proprietor: The University of Tokushima, Tokushima-shi, Tokushima 770-8501 (JP)
(72) Inventor: YASUTOMO, Koji, Tokushima-shi Tokushima 770-8503 (JP)
(74) Representative: Duckworth, Timothy John
(86) International application number: PCT/JP2011/057432
(87) International publication number: WO 2011/118804

(56) References cited:
- EP-A1- 2 011 869
- WO-A2-2008/017828
- JP-A- 8 503 222
- JP-A- 2004 533 449
- ITOH KUNIHIKO ET AL: "Phage display cloning and characterization of monoclonal antibody genes and recombinant Fab fragment against the CD98 oncoprotein", JAPANESE JOURNAL OF CANCER RESEARCH, JAPANESE CANCER ASSOCIATION, TOKYO, JP, vol. 92, no. 12, 1 December 2001 (2001-12-01), pages 1313-1321, XP002476421, ISSN: 0910-5050
- DIAZ L. ET AL.: 'Monocyte-dependent regulation of T lymphocyte activation through CD98.' INTERNATIONAL IMMUNOLOGY vol. 9, no. 9, 1997, pages 1221 - 1231, XP003019767
- KOMADA H. ET AL.: 'Possible activation of murine T lymphocyte through CD98 is independent of interleukin 2/interleukin 2 receptor system.' BIOMEDICAL RESEARCH vol. 27, 2006, pages 61 - 67
- ITOH K. ET AL.: 'Identification of cell proliferation-associated epitope on CD98 oncoprotein using phage display random peptide library.' CANCER SCI. vol. 98, no. 11, 2007, pages 1696 - 1700, XP055054055
- WOODHEAD V. E. ET AL.: 'Novel molecular mechanisms of dendritic cell-induced T cell activation.' INTERNATIONAL IMMUNOLOGY vol. 12, 2000, pages 1051 - 1061
- CANTOR J. ET AL.: 'CD98hc facilitates B cell proliferation and adaptive humoral immunity.' NATURE IMMUNOLOGY vol. 10, 2009, pages 412 - 419

### SEQUENCE LISTING

<110> The University of Tokushima
<120> A NOVEL ANTI-CD98 ANTIBODY AND USE THEREOF
<130> 091682
<150> JP2010-072966
   <151> 2010-03-26
<150> JP2010-200599
   <151> 2010-09-08
<150> JP2010-273455
   <151> 2010-12-08
<160> 28
<170> Patent In version 3.4
<210> 1
   <211> 508
   <212> DNA
   <213> Mus musculus
<220>
   <221> CDS
   <222> (1)..(507)
<400> 1
   atg gac acc agg ctc agc ttg gtt ttc ctt gtc ctt ttc ata aaa ggt    48
   Met Asp Thr Arg Leu Ser Leu Val Phe Leu Val Leu Phe Ile Lys Gly
   1               5                   10                  15
   gtc cag tgt gag gtg cag ctg gtg gag tct ggg gga ggc tta gtg cag    96
   Val Gln Cys Glu Val Gln Leu Val Glu Ser Gly Gly Gly Leu Val Gln
               20                  25                  30
   cct gga agg tcc ctg aaa ctc tcc tgt gca gcc tca gga ttc act ttc    144
   Pro Gly Arg Ser Leu Lys Leu Ser Cys Ala Ala Ser Gly Phe Thr Phe
           35                  40                  45
   agt gac tat tac atg gcc tgg gtc cgc cag gct cca aag aag ggt ctg    192
   Ser Asp Tyr Tyr Met Ala Trp Val Arg Gln Ala Pro Lys Lys Gly Leu
       50                  55                  60
   gag tgg gtc gca tcc att agt tat gag ggt agt agt att tac tat gga    240
   Glu Trp Val Ala Ser Ile Ser Tyr Glu Gly Ser Ser Ile Tyr Tyr Gly
   65                  70                  75                  80
   gac tcc gtg aag ggc cga gtc act atc tcc aga gat aat gca aaa agc    288
   Asp Ser Val Lys Gly Arg Val Thr Ile Ser Arg Asp Asn Ala Lys Ser
                   85                  90                  95
   acc cta tac ctg caa atg aac agt ctg agg tct gag gac acg gcc act    336
   Thr Leu Tyr Leu Gln Met Asn Ser Leu Arg Ser Glu Asp Thr Ala Thr
               100                 105                 110
   tat tac tgt gca aga cgg gga tac tat gga tat aag ccc ttc gat tac    384
   Tyr Tyr Cys Ala Arg Arg Gly Tyr Tyr Gly Tyr Lys Pro Phe Asp Tyr
           115                 120                 125
   tgg ggc caa gga gtc atg gtc aca gtc tcc tca gcc caa aca aca gcc    432
   Trp Gly Gln Gly Val Met Val Thr Val Ser Ser Ala Gln Thr Thr Ala
       130                 135                 140
   cca tct gtc tat cca ctg gct cct gga tgt ggt gat aca acc agc tcc    480
   Pro Ser Val Tyr Pro Leu Ala Pro Gly Cys Gly Asp Thr Thr Ser Ser
   145                 150                 155                 160
   acg gtg act ctg gga tgc ctg gtc aag g                              508
   Thr Val Thr Leu Gly Cys Leu Val Lys
                   165
<210> 2
   <211> 169
   <212> PRT
   <213> Mus musculus
<400> 2
   Met Asp Thr Arg Leu Ser Leu Val Phe Leu Val Leu Phe Ile Lys Gly
   1               5                   10                  15
   Val Gln Cys Glu Val Gln Leu Val Glu Ser Gly Gly Gly Leu Val Gln
               20                  25                  30
   Pro Gly Arg Ser Leu Lys Leu Ser Cys Ala Ala Ser Gly Phe Thr Phe
           35                  40                  45
   Ser Asp Tyr Tyr Met Ala Trp Val Arg Gln Ala Pro Lys Lys Gly Leu
       50                  55                  60
   Glu Trp Val Ala Ser Ile Ser Tyr Glu Gly Ser Ser Ile Tyr Tyr Gly
   65                  70                  75                  80
   Asp Ser Val Lys Gly Arg Val Thr Ile Ser Arg Asp Asn Ala Lys Ser
                   85                  90                  95
   Thr Leu Tyr Leu Gln Met Asn Ser Leu Arg Ser Glu Asp Thr Ala Thr
               100                 105                 110
   Tyr Tyr Cys Ala Arg Arg Gly Tyr Tyr Gly Tyr Lys Pro Phe Asp Tyr
           115                 120                 125
   Trp Gly Gln Gly Val Met Val Thr Val Ser Ser Ala Gln Thr Thr Ala
       130                 135                 140
   Pro Ser Val Tyr Pro Leu Ala Pro Gly Cys Gly Asp Thr Thr Ser Ser
   145                 150                 155                 160
   Thr Val Thr Leu Gly Cys Leu Val Lys
                   165
<210> 3
   <211> 393
   <212> DNA
   <213> Mus musculus
<220>
   <221> CDS
   <222> (1)..(393)
<400> 3
   gtc acc atc gaa tgt cga gca agt gaa gac att tac aat ggt tta gca       48
   Val Thr Ile Glu Cys Arg Ala Ser Glu Asp Ile Tyr Asn Gly Leu Ala
   1               5                   10                  15
   tgg tat cag cag aaa cca ggg aaa tct cct cag ctc ctg atc tat aat       96
   Trp Tyr Gln Gln Lys Pro Gly Lys Ser Pro Gln Leu Leu Ile Tyr Asn
               20                  25                  30
   ata gat aac ttg cat act ggg gtc cca tca cgg ttc agt ggc agt gga      144
   Ile Asp Asn Leu His Thr Gly Val Pro Ser Arg Phe Ser Gly Ser Gly
           35                  40                  45
   tct ggt aca cag tat tct ctc aag ata aac agc cta caa tcg gag gat      192
   Ser Gly Thr Gln Tyr Ser Leu Lys Ile Asn Ser Leu Gln Ser Glu Asp
       50                  55                  60
   gtc gca agt tat ttc tgt cag cag tat tac act tat gcg tac acg ttt      240
   Val Ala Ser Tyr Phe Cys Gln Gln Tyr Tyr Thr Tyr Ala Tyr Thr Phe
   65                  70                  75                  80
   gga gct ggg acc aag ctg gaa ctg aaa cgg gct gat gct gca cca act      288
   Gly Ala Gly Thr Lys Leu Glu Leu Lys Arg Ala Asp Ala Ala Pro Thr
                   85                  90                  95
   gta tcc atc ttc cca cca tcc atg gaa cag tta aca tct gga ggt gcc      336
   Val Ser Ile Phe Pro Pro Ser Met Glu Gln Leu Thr Ser Gly Gly Ala
               100                 105                 110
   aca gtc gtg tgc ttc gtg aac aac ttc tat ccc aga gac atc agt gtc      384
   Thr Val Val Cys Phe Val Asn Asn Phe Tyr Pro Arg Asp Ile Ser Val
           115                 120                 125
   aag tgg aaa                                                          393
   Lys Trp Lys
       130
<210> 4
   <211> 131
   <212> PRT
   <213> Mus musculus
<400> 4
   Val Thr Ile Glu Cys Arg Ala Ser Glu Asp Ile Tyr Asn Gly Leu Ala
   1               5                   10                  15
   Trp Tyr Gln Gln Lys Pro Gly Lys Ser Pro Gln Leu Leu Ile Tyr Asn
               20                  25                  30
   Ile Asp Asn Leu His Thr Gly Val Pro Ser Arg Phe Ser Gly Ser Gly
           35                  40                  45
   Ser Gly Thr Gln Tyr Ser Leu Lys Ile Asn Ser Leu Gln Ser Glu Asp
       50                  55                  60
   Val Ala Ser Tyr Phe Cys Gln Gln Tyr Tyr Thr Tyr Ala Tyr Thr Phe
   65                  70                  75                  80
   Gly Ala Gly Thr Lys Leu Glu Leu Lys Arg Ala Asp Ala Ala Pro Thr
                   85                  90                  95
   Val Ser Ile Phe Pro Pro Ser Met Glu Gln Leu Thr Ser Gly Gly Ala
               100                 105                 110
   Thr Val Val Cys Phe Val Asn Asn Phe Tyr Pro Arg Asp Ile Ser Val
           115                 120                 125
   Lys Trp Lys
       130
<210> 5
   <211> 540
   <212> DNA
   <213> Homo sapiens
<220>
   <221> CDS
   <222> (1)..(540)
<400> 5
   cac aga cta ctc acc atg gac atc agg ctc agc ttg gct ttc ctt gtc      48
   His Arg Leu Leu Thr Met Asp Ile Arg Leu Ser Leu Ala Phe Leu Val
   1               5                   10                  15
   ctt ttc ata aaa ggt gtc cag tgt gag gtg cag ctg gtg gag tct ggg      96
   Leu Phe Ile Lys Gly Val Gln Cys Glu Val Gln Leu Val Glu Ser Gly
               20                  25                  30
   gga ggc tta gtg cag cct gga cgg tcc atg aaa ctc tcc tgt gca gcc     144
   Gly Gly Leu Val Gln Pro Gly Arg Ser Met Lys Leu Ser Cys Ala Ala
           35                  40                  45
   tca gga ttc act ttc agt aac tat tac atg gcc tgg gtc cgc cag gct     192
   Ser Gly Phe Thr Phe Ser Asn Tyr Tyr Met Ala Trp Val Arg Gln Ala
       50                  55                  60
   cca acg aag ggt ctg gag tgg gtc gca tcc att agt gct ggt ggt gtt     240
   Pro Thr Lys Gly Leu Glu Trp Val Ala Ser Ile Ser Ala Gly Gly Val
   65                  70                  75                  80
   aac aat tac tat cga gac tcc atg aag ggc cga ttc act atc tcc aga     288
   Asn Asn Tyr Tyr Arg Asp Ser Met Lys Gly Arg Phe Thr Ile Ser Arg
                   85                  90                  95
   gat aat gca aaa agc acc cta tac ctg caa atg gac ggt ctg agg tct     336
   Asp Asn Ala Lys Ser Thr Leu Tyr Leu Gln Met Asp Gly Leu Arg Ser
               100                 105                 110
   gag gac acg gcc act tat tac tgt gca cga ctt gtg aat aat tta ggt     384
   Glu Asp Thr Ala Thr Tyr Tyr Cys Ala Arg Leu Val Asn Asn Leu Gly
           115                 120                 125
   aca aac gtg ttt cct gac tgg ggc caa ggc act ctg gtc act gtc tct     432
   Thr Asn Val Phe Pro Asp Trp Gly Gln Gly Thr Leu Val Thr Val Ser
       130                 135                 140
   tta gcc caa aca aca gcc cca tct gtc tat cca ctg gct cct gga tgt     480
   Leu Ala Gln Thr Thr Ala Pro Ser Val Tyr Pro Leu Ala Pro Gly Cys
   145                 150                 155                 160
   ggt gat aca acc agc tcc acg gtg act ctg gga tgc ctg gtc aag ggc     528
   Gly Asp Thr Thr Ser Ser Thr Val Thr Leu Gly Cys Leu Val Lys Gly
                   165                 170                 175
   tat ttc cct gag                                                     540
   Tyr Phe Pro Glu
               180
<210> 6
   <211> 180
   <212> PRT
   <213> Homo sapiens
<400> 6
   His Arg Leu Leu Thr Met Asp Ile Arg Leu Ser Leu Ala Phe Leu Val
   1 5 10 15
   Leu Phe Ile Lys Gly Val Gln Cys Glu Val Gln Leu Val Glu Ser Gly
   20 25 30
   Gly Gly Leu Val Gln Pro Gly Arg Ser Met Lys Leu Ser Cys Ala Ala
   35 40 45
   Ser Gly Phe Thr Phe Ser Asn Tyr Tyr Met Ala Trp Val Arg Gln Ala
   50 55 60
   Pro Thr Lys Gly Leu Glu Trp Val Ala Ser Ile Ser Ala Gly Gly Val
   65 70 75 80
   Asn Asn Tyr Tyr Arg Asp Ser Met Lys Gly Arg Phe Thr Ile Ser Arg
   85 90 95
   Asp Asn Ala Lys Ser Thr Leu Tyr Leu Gln Met Asp Gly Leu Arg Ser
   100 105 110
   Glu Asp Thr Ala Thr Tyr Tyr Cys Ala Arg Leu Val Asn Asn Leu Gly
   115 120 125
   Thr Asn Val Phe Pro Asp Trp Gly Gln Gly Thr Leu Val Thr Val Ser
   130 135 140
   Leu Ala Gln Thr Thr Ala Pro Ser Val Tyr Pro Leu Ala Pro Gly Cys
   145 150 155 160
   Gly Asp Thr Thr Ser Ser Thr Val Thr Leu Gly Cys Leu Val Lys Gly
   165 170 175
   Tyr Phe Pro Glu
   180
<210> 7
   <211> 443
   <212> DNA
   <213> Homo sapiens
<220>
   <221> CDS
   <222> (1)..(441)
<400> 7
   gct gtg tca gcg gga gag gcg gtc act ata aac tgc aag tcc agt cag        48
   Ala Val Ser Ala Gly Glu Ala Val Thr Ile Asn Cys Lys Ser Ser Gln
   1               5                   10                  15
   agt ctt tta tcc agt gga aac caa aag aac tac ttg gcc tgg tac cag        96
   Ser Leu Leu Ser Ser Gly Asn Gln Lys Asn Tyr Leu Ala Trp Tyr Gln
               20                  25                  30
   cag aaa cca ggg cag tct cct aaa ctg ctg atc tac tgg gca tct act       144
   Gln Lys Pro Gly Gln Ser Pro Lys Leu Leu Ile Tyr Trp Ala Ser Thr
           35                  40                  45
   agg caa tct ggt gtc cct gat cgc ttc ata ggc agt gga tct ggg aca       192
   Arg Gln Ser Gly Val Pro Asp Arg Phe Ile Gly Ser Gly Ser Gly Thr
       50                  55                  60
   gac ttc act ctg acc atc agc ggt gtg cag gca gaa gat ctg gca gtt       240
   Asp Phe Thr Leu Thr Ile Ser Gly Val Gln Ala Glu Asp Leu Ala Val
   65                  70                  75                  80
   tat tac tgt cag cag tat aag gaa att ccg ctc acg ttc ggt tct ggg       288
   Tyr Tyr Cys Gln Gln Tyr Lys Glu Ile Pro Leu Thr Phe Gly Ser Gly
                   85                  90                  95
   acc aaa ctg gag atc aaa cgg gct gat gct gca cca act gta tcc atc       336
   Thr Lys Leu Glu Ile Lys Arg Ala Asp Ala Ala Pro Thr Val Ser Ile
               100                 105                 110
   ttc cca cca tcc atg gaa cag tta aca tct gga ggt gcc aca gtc gtg       384
   Phe Pro Pro Ser Met Glu Gln Leu Thr Ser Gly Gly Ala Thr Val Val
           115                 120                 125
   tgc ttc gtg aac aac ttc tat ccc aga gac atc agt gtc aag tgg aag       432
   Cys Phe Val Asn Asn Phe Tyr Pro Arg Asp Ile Ser Val Lys Trp Lys
       130                 135                 140
   att gat ggc ag                                                        443
   Ile Asp Gly
   145
<210> 8
   <211> 147
   <212> PRT
   <213> Homo sapiens
<400> 8
   Ala Val Ser Ala Gly Glu Ala Val Thr Ile Asn Cys Lys Ser Ser Gln
   1               5                   10                  15
   Ser Leu Leu Ser Ser Gly Asn Gln Lys Asn Tyr Leu Ala Trp Tyr Gln
               20                  25                  30
   Gln Lys Pro Gly Gln Ser Pro Lys Leu Leu Ile Tyr Trp Ala Ser Thr
           35                  40                  45
   Arg Gln Ser Gly Val Pro Asp Arg Phe Ile Gly Ser Gly Ser Gly Thr
       50                  55                  60
   Asp Phe Thr Leu Thr Ile Ser Gly Val Gln Ala Glu Asp Leu Ala Val
   65                  70                  75                  80
   Tyr Tyr Cys Gln Gln Tyr Lys Glu Ile Pro Leu Thr Phe Gly Ser Gly
                   85                  90                  95
   Thr Lys Leu Glu Ile Lys Arg Ala Asp Ala Ala Pro Thr Val Ser Ile
               100                 105                 110
   Phe Pro Pro Ser Met Glu Gln Leu Thr Ser Gly Gly Ala Thr Val Val
           115                 120                 125
   Cys Phe Val Asn Asn Phe Tyr Pro Arg Asp Ile Ser Val Lys Trp Lys
       130                 135                 140
   Ile Asp Gly
   145
<210> 9
   <211> 26
   <212> DNA
   <213> Artificial
<220>
   <223> primer (gamma chain)
<400> 9
   cggaattcag gtsmarctgc agsagt 26
<210> 10
   <211> 29
   <212> DNA
   <213> Artificial
<220>
   <223> primer (gamma chain)
<400> 10
   gcggatccgg acagggctcc agagttcca 29
<210> 11
   <211> 30
   <212> DNA
   <213> Artificial
<220>
   <223> primer (kappa chain)
<400> 11
   gcgaattcga cayyvwgmtg achcagtctc 30
<210> 12
   <211> 31
   <212> DNA
   <213> Artificial
<220>
   <223> primer (kappa chain)
<400> 12
   cgcgaagctt tcagtaacac tgtycaggac a 31
<210> 13
   <211> 5
   <212> PRT
   <213> Mus muscillus
<400> 13
   Asp Tyr Tyr Met Ala
   1 5
<210> 14
   <211> 17
   <212> PRT
   <213> Mus musculus
<400> 14
   Ser Ile Ser Tyr Glu Gly Ser Ser Ile Tyr Tyr Gly Asp Ser Val Lys
   1 5 10 15
   Gly
<210> 15
   <211> 11
   <212> PRT
   <213> Mus musculus
<400> 15
   Arg Gly Tyr Tyr Gly Tyr Lys Pro Phe Asp Tyr
   1 5 10
<210> 16
   <211> 11
   <212> PRT
   <213> Mus musculus
<400> 16
   Arg Ala Ser Glu Asp Ile Tyr Asn Gly Leu Ala
   1 5 10
<210> 17
   <211> 7
   <212> PRT
   <213> Mus musculus
<400> 17
   Asn Ile Asp Asn Leu His Thr
   1 5
<210> 18
   <211> 9
   <212> PRT
   <213> Mus musculus
<400> 18
   Gln Gln Tyr Tyr Thr Tyr Ala Tyr Thr
   1 5
<210> 19
   <211> 5
   <212> PRT
   <213> Homo sapiens
<400> 19
   Asn Tyr Tyr Met Ala
   1 5
<210> 20
   <211> 17
   <212> PRT
   <213> Homo sapiens
<400> 20
   Ser Ile Ser Ala Gly Gly Val Asn Asn Tyr Tyr Arg Asp Ser Met Lys
   1 5 10 15
   Gly
<210> 21
   <211> 12
   <212> PRT
   <213> Homo sapiens
<400> 21
   Leu Val Asn Asn Leu Gly Thr Asn Val Phe Pro Asp
   1 5 10
<210> 22
   <211> 17
   <212> PRT
   <213> Homo sapiens
<400> 22
   Lys Ser Ser Gln Ser Leu Leu Ser Ser Gly Asn Gln Lys Asn Tyr Leu
   1 5 10 15
   Ala
<210> 23
   <211> 7
   <212> PRT
   <213> Homo sapiens
<400> 23
   Trp Ala Ser Thr Arg Gln Ser
   1 5
<210> 24
   <211> 9
   <212> PRT
   <213> Homo sapiens
<400> 24
   Gln Gln Tyr Lys Glu Ile Pro Leu Thr
   1 5
<210> 25
   <211> 129
   <212> PRT
   <213> Rattus norvegicus
<400> 25
   Lys Gly Val Gln Cys Glu Val Gln Leu Val Glu Ser Gly Gly Gly Leu
   1 5 10 15
   Val Gln Pro Gly Arg Ser Leu Lys Leu Ser Cys Ala Ala Ser Gly Phe
   20 25 30
   Thr Phe Ser Asp Tyr Tyr Met Ala Trp Val Arg Gln Ala Pro Thr Lys
   35 40 45
   Gly Leu Glu Trp Val Ala Ser Ile Ser Tyr Asp Gly Gly Ala Thr Tyr
   50 55 60
   Tyr Arg Asp Ser Val Lys Gly Arg Phe Thr Ile Ser Arg Asp Asn Ala
   65 70 75 80
   Lys Ser Ser Leu Tyr Leu Gln Val Asp Gly Leu Arg Ser Glu Asp Thr
   85 90 95
   Ala Thr Tyr Tyr Cys Thr Thr Gly Ser Ser Phe Thr Gly Gly Tyr Trp
   100 105 110
   Gly Gln Gly Val Met Val Thr Val Ser Ser Ala Gln Thr Thr Ala Pro
   115 120 125
   Ser
<210> 26
   <211> 140
   <212> PRT
   <213> Homo sapiens
<400> 26
   Ser Thr Thr Met Lys His Leu Trp Phe Phe Leu Leu Leu Val Ala Ala
   1               5                   10                  15
   Pro Arg Trp Val Leu Ser Gln Leu Gln Leu Gln Glu Ser Gly Pro Gly
               20                  25                  30
   Leu Val Lys Pro Ser Glu Thr Leu Ser Leu Thr Cys Thr Val Ser Gly
           35                  40                  45
   Gly Ser Ile Ser Ser Ser Ser Tyr Tyr Trp Gly Trp Arg Gln Pro Pro
       50                  55                  60
   Gly Lys Leu Glu Trp Ile Gly Ser Ile Tyr Tyr Ser Gly Ser Thr Tyr
   65                  70                  75                  80
   Tyr Asn Pro Ser Leu Lys Ser Arg Val Thr Ile Ser Val Asp Thr Ser
                   85                  90                  95
   Lys Asn Gln Phe Ser Leu Lys Leu Ser Ser Val Thr Ala Ala Asp Thr
               100                 105                 110
   Ala Val Tyr Tyr Cys Ala Arg Gln Gly Thr Gly Leu Ala Leu Phe Asp
           115                 120                 125
   Tyr Trp Gly Gln Gly Thr Leu Val Thr Val Ser Ser
       130                 135                 140
<210> 27
   <211> 90
   <212> PRT
   <213> Rattus norvegicus
<400> 27
   Ala Thr Ile Ser Cys Arg Ala Ser Glu Ser Val Arg Ser Ser Met His
   1 5 10 15
   Trp Tyr Gln Gln Lys Ser Gly Gln Gln Pro Lys Leu Leu Ile Tyr Gly
   20 25 30
   Ala Ser Asn Leu Ala Ser Gly Val Pro Ala Arg Phe Ser Gly Ser Gly
   35 40 45
   Ser Gly Thr Asp Phe Thr Phe Thr Ile Asp Pro Val Glu Ala Asp Asp
   50 55 60
   Ile Ala Thr Tyr Tyr Cys Gln Gln Ser Arg Asn Asp Pro Tyr Thr Phe
   65 70 75 80
   Gly Ala Gly Thr Lys Leu Glu Leu Lys Arg
   85 90
<210> 28
   <211> 99
   <212> PRT
   <213> Homo sapiens
<400> 28
   Leu Ser Leu Ser Pro Gly Glu Arg Ala Thr Leu Ser Cys Arg Ala Ser
   1 5 10 15
   Gln Ser Val Ser Ser Ser Phe Leu Ala Trp Tyr Gln Gln Lys Pro Gly
   20 25 30
   Gln Ala Pro Arg Leu Leu Ile Tyr Gly Ala Ser Ser Arg Ala Thr Gly
   35 40 45
   Ile Pro Asp Arg Phe Ser Gly Ser Gly Ser Gly Thr Asp Phe Thr Leu
   50 55 60
   Thr Ile Ser Arg Leu Glu Pro Glu Asp Phe Ala Val Tyr Tyr Cys Gln
   65 70 75 80
   Gln Tyr Gly Ser Ser Pro Cys Phe Thr Phe Gly Pro Gly Thr Lys Val
   85 90 95
   Asp Ile Lys

## Description

### Technical Field

The present invention relates to a novel anti-CD98 antibody.

### Background Art

CD98 is an about 120kDa heterodimer glycoprotein widely expressed on a cellular membrane, and is considered to be related to amino acid transport, and cell fusion associated with increased cellular membrane expression and integrin mediated adhesion. As the structure of CD98, a heterodimer is constituted by about 80kDa type II transmembrane protein CD98hc (heavy chain: 4F2hc, SLC3A2) and one of the 6 kinds of about 40kDa light chains (LAT1, LAT2, y+LAT1, y+LAT2, xCT, asc) by a disulfide bond and functions as an amino acid transporter. The heavy chain of CD98 (CD98hc) functions to move the dimer to a cellular membrane, and light chain (1c) is considered to function as a transporter.

CD98hc was first discovered as a T cell activated antigen, and is a glycoprotein having various functions. As its function, it is highly expressed in growing lymphocytes, tumor cells, and other cells with high proliferative capacity, and plays a key role in integrin dependent signaling that promotes tumor formation.

The amino acid transport system involving CD98 is constituted with one of the 6 kinds of transporters (about 40kDa light chains), reflecting the diversity of amino acid molecule to be the substrate, and classified into various transport systems by transport substrate selectivity and Na⁺ dependency. For example, it forms a disulfide bond with a particular transporter (LAT1, LAT2, y+LAT1, y+LAT2, xCT, asc) corresponding to the neutral amino acid transport system L, compact neutral amino acid transport system asc, neutral and basic amino acid transport system y+L, cystine, and basic and neutral amino acid transport system x-c. CD98 heavy chain is present in the cellular membrane of epithelial cells on the blood vessel side, and functions to deliver the above-mentioned transporter to the cellular membrane of epithelial cell on the blood vessel side.

In addition, CD98 is known to be highly expressed in various cancer cells. The reason therefor is considered to be overexpression of neutral amino acid transporter, since cancer cells in need of cell proliferation require active uptake of essential amino acids which are necessary for the growth. For example, L-type amino acid transporter 1 (LAT1) was cloned as an amino acid transporter that is specifically and highly expressed in cancer cell (Kanai et al., J. Biol. Chem. (1998), 273, 23629-23632). LAT1 forms a complex with CD98 heavy chain and transports neutral amino acids having large side chains, such as leucine, valine, phenylalanine, tyrosine, tryptophan, methionine, histidine and the like in a sodium ion-independent manner. It is known that LAT1 is poorly or not expressed in most normal tissue except for the brain, placenta, bone marrow and testis, but its expression increases together with CD98 in tissues of human malignant tumors such as colorectal cancer, gastric cancer, breast cancer, pancreatic cancer, kidney cancer, laryngeal cancer, esophagus cancer, lung cancer and the like (non-patent document 1).

Therefore, it was considered that reduced expression of CD98 heavy chain and light chain (LAT1) and suppression of amino acid uptake might suppress tumor growth. Suppression of tumor growth by reduced expression of light chain (LAT1) has been reported in cancer transplantation mouse model (patent document 1). Therefore, suppression of LAT1 activity was considered to be a promising treatment method of cancer.

In addition, a cancer cell suppressive effect by the use of anti-CD98 antibody has been reported in cancer-transplanted mouse model, for the suppression of amino acid transport by CD98 (complex of heavy chain and light chain) (patent documents 2, 3). Thus, various attempts have been made to use anti-CD98 antibody as an anti-cancer agent. However, efforts relating to use other than that as an anti-cancer agent have been scarcely made as the situation stands.
Patent documents 4 and 5 and non-patent document 2 disclose anti-CD98 antibodies.

### [Document List]

### [patent documents]

patent document 1: JP-A-2000-157286
patent document 2: JP-A-2009-189376
patent document 3: WO2007/114496
Patent document 4: EP 2011869 A1
Patent document 5: WO 2008/017828 A2

### [non-patent document]

non-patent document 1: Yanagida et al., Biochem. Biophys. Acta, (2001), 1514, 291-302
Non-patent document 2: Itoh et al., Japanese Journal of Cancer Research, (2001), 92, 1313-1321

### SUMMARY OF THE INVENTION

### Problems to be Solved by the Invention

The present invention provides a new anti-CD98 antibody, and the antibody for use in the prophylaxis or treatment of autoimmune diseases such as type 1 diabetes, rheumatoid arthritis, ulcerative colititis, psoriasis, Chrone's disease, systemic lupus erythematosus or Basedow's disease. Means of Solving the Problems

Since CD98 knockout mouse dies during early fetal life, CD98 is considered to play an important role in amino acid transport. Therefore, the present inventor has studied inhibition of amino acid uptake into cells to promote, in diabetes diseases, sugar uptake into cells and consumption.

In this study, the present inventor has clarified that CD98 (complex of heavy chain and light chain) having an amino acid transport activity functions to positively control T cell activation. To further advance their research, he has prepared an anti-CD98 antibody having a specific binding ability to the heavy chain of CD98. Characteristically, different from the anti-CD98 antibody of patent document 2, the anti-CD98 antibody of the present invention does not at all influence amino acid transport, but remarkably suppresses activation of T lymphocytes that destroy pancreatic B cell. Furthermore, the anti-CD98 antibody of the present invention characteristically suppresses cell adhesion via fibronectin. Since fibronectin is an adhesion factor necessary for T cell to adhere to other cells to exhibit its function and the anti-CD98 antibody of the present invention suppresses cell adhesion mediated by fibronectin, it was considered that adhesion of T cell to pancreatic B cell can be suppressed, as a result of which destruction of pancreatic B cell by T cell can be suppressed.

Moreover, to find the effect of the anti-CD98 antibody of the present invention, experiments using various animal models were conducted to find that the present antibody not only suppresses progression of type 1 diabetes but also can treat same. That is, the present antibody has been shown to be highly effective for type 1 diabetes based on apoptosis of pancreatic B cell, for which no appropriate treatment method has been available heretofore other than a supplementation therapy of insulin.

Furthermore, it has been clarified that the anti-CD98 antibody of the present invention can also suppress progression of rheumatoid arthritis. These findings have shown that the anti-CD98 antibody of the present invention is useful as a therapeutic drug for autoimmune diseases. The present inventor has completed the present invention based on the above findings.

Accordingly, the present invention provides the following.
[1] An anti-CD98 antibody or a functional antigen-binding fragment thereof, which comprises the sequence shown by SEQ ID NO: 19 as CDR1 of a heavy chain, the sequence shown by SEQ ID NO: 20 as CDR2 of a heavy chain, and the sequence shown by SEQ ID NO: 21 as CDR3 of a heavy chain, the sequence shown by SEQ ID NO: 22 as CDR1 of a light chain, the sequence shown by SEQ ID NO: 23 as CDR2 of a light chain, and the sequence shown by SEQ ID NO: 24 as CDR3 of a light chain.
[2] The anti-CD98 antibody or a functional antigen-binding fragment thereof according to [1], which comprises 2 sequences of SEQ ID NOs: 6 and 8 as a heavy chain variable region and a light chain variable region, respectively.
[3] The anti-CD98 antibody or a functional antigen-binding fragment thereof according to [1] or [2], wherein the anti-CD98 antibody is produced by NITE BP-1007.
[4] The anti-CD98 antibody or a functional antigen-binding fragment thereof according to any one of [1] to [3], wherein the class of the antibody heavy chain constant region is IgG.
[5] The anti-CD98 antibody or a functional antigen-binding fragment thereof according to any one of [1] to [4], wherein the aforementioned functional fragment is selected from the group consisting of heavy chain and light chain variable regions (V_{H} and V_{L}), Fab, Fab' , F(ab')₂, Fv, scFv, sdFv of the antibody and combinations thereof.
[6] An anti-CD98 antibody or a functional antigen-binding fragment thereof, which comprises the sequence shown by SEQ ID NO: 13 as CDR1 of a heavy chain, the sequence shown by SEQ ID NO: 14 as CDR2 of a heavy chain, and the sequence shown by SEQ ID NO: 15 as CDR3 of a heavy chain, the sequence shown by SEQ ID NO: 16 as CDR1 of a light chain, the sequence shown by SEQ ID NO: 17 as CDR2 of a light chain, and the sequence shown by SEQ ID NO: 18 as CDR3 of a light chain.
[7] The anti-CD98 antibody or a functional antigen-binding fragment thereof according to [6], which comprises 2 sequences of SEQ ID NOs: 2 and 4 as a heavy chain variable region and a light chain variable region, respectively.
[8] The anti-CD98 antibody or a functional antigen-binding fragment thereof according to [6] or [7], wherein the class of the antibody heavy chain constant region is IgG.
[9] The anti-CD98 antibody or a functional antigen-binding fragment thereof according to any one of [6] to [8], wherein the aforementioned functional fragment is selected from the group consisting of heavy chain and light chain variable regions (V_{H} and V_{L}), Fab, Fab', F(ab')₂, Fv, scFv, sdFv of the antibody and combinations thereof.
[10] The anti-CD98 antibody or a functional antigen-binding fragment thereof according to any one of [6] to [9], wherein the anti-CD98 antibody is produced by NITE BP-964.
[11] A pharmaceutical composition comprising the anti-CD98 antibody or a functional antigen-binding fragment thereof according to any one of [1] to [10] as an active ingredient.
[12] The anti-CD98 antibody or a functional antigen-binding fragment thereof according to any one of [1] to [10], for use in the prophylaxis or treatment of an autoimmune disease.
[13] The anti-CD98 antibody or a functional antigen-binding fragment thereof for use according to [12], wherein the autoimmune disease is type 1 diabetes, rheumatoid arthritis, ulcerative colitis, psoriasis, Crohn's disease, systemic lupus erythematosus or Basedow's disease.

### Effect of the Invention

The present invention provides a novel anti-CD98 antibody for treating an autoimmune disease. The anti-CD98 antibody of the present invention is particularly effective for type 1 diabetes and rheumatoid arthritis, for which there is no effective treatment. Furthermore, since the anti-CD98 antibody of the present invention effectively suppresses T cell activation but does not affect amino acid uptake by CD98, it is an antibody having less impact on the energy metabolism and causing less side effects. With the functional features of the anti-CD98 antibody of the present invention, when the anti-CD98 antibody of the present invention is used for the treatment of autoimmune diseases, for example, treatment of diabetes, the exhaustion and depletion of pancreatic B cells can be suppressed, and particularly, progression of type 1 diabetes can be suppressed, thus enabling the prophylaxis and treatment of type 1 diabetes. When the autoimmune disease is rheumatoid arthritis, for example, the onset and progression of arthritis can be suppressed by the use of the anti-CD98 antibody of the present invention, thus enabling the prophylaxis and treatment of rheumatoid arthritis. From the above, a therapeutic agent for an autoimmune disease containing the anti-CD98 antibody of the present invention as an active ingredient is extremely useful for the treatment of autoimmune diseases (for example, type 1 diabetes and rheumatoid arthritis), for which there is no particularly appropriate treatment.

### Brief Description of the Drawings

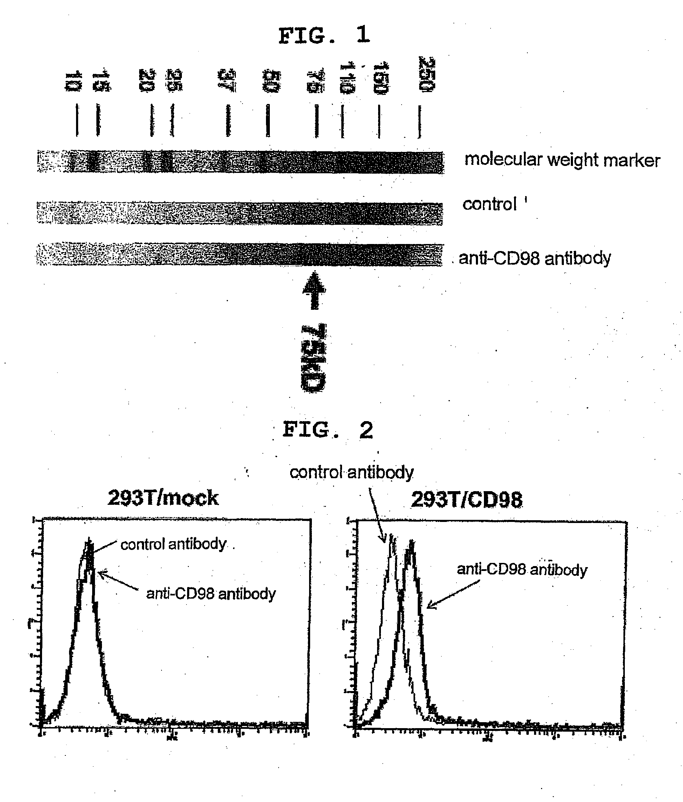

Fig. 1 shows the results of evaluation of the molecular weight by electrophoresis of an isolated recognition molecule with which the anti-CD98 antibody of the present invention reacts. An anti-mouse CD98 antibody (mCD98Ab) was added to a solution wherein mouse spleen cells were solubilized with NP40, and stood for 2 hr at 4°C. Then, protein G agarose was added and the mixture was cultured with shaking for 2 hr at 4°C. Free anti-CD98 antibody was removed by washing, a sample buffer was added, and the mixture was treated at 98°C for 3 min, and electrophoresed on polyacrylamide gel. The gel used for electrophoresis was stained silver. As a result, color development of stained silver was found at about 75kDa which is the molecular weight of CD98 heavy chain.

Fig. 2 shows that the mCD98Ab of the present invention recognizes and reacts with CHO cell that expresses mouse CD98 (right Figure). Mouse CD98 gene incorporated into pcDNA3.1 or pcDNA3.1 alone was used for transfection of CHO cell by an electroporation method. To select a cell having CD98 gene transfected into the genome of CHO cell, drug selection was performed using G418, and a drug resistance cell was isolated. The cell was stained with rat immunoglobulin G or mCD98Ab, and washed to remove free antibody and stained with PE-labeled anti-rat IgG. As a result, mCD98Ab did not react with a cell free of CD98 gene introduction, and reacted with a cell transfected with CD98 gene. The left Figure shows the results of reaction with the cell transfected with pcDNA3.1 alone and the right Figure shows the results of reaction with the cell after introduction and expression of mouse CD98 gene.

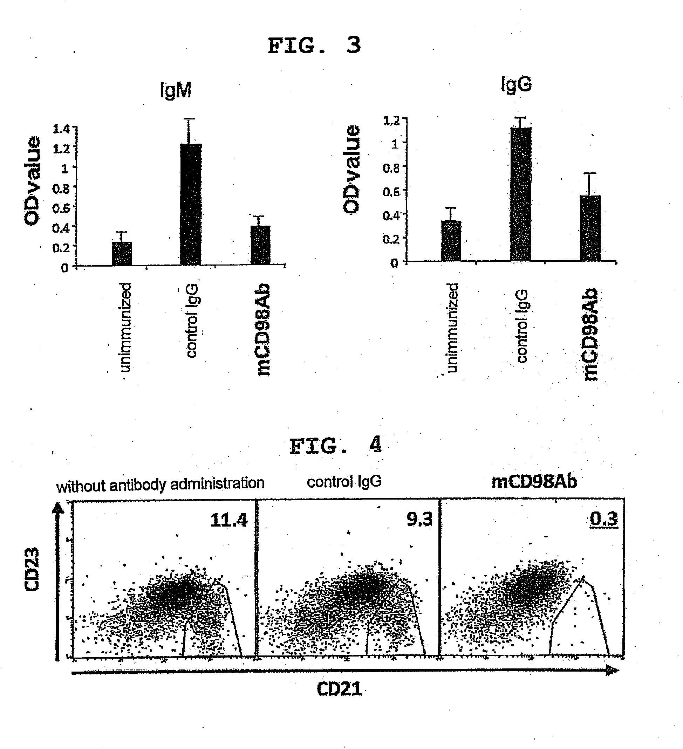

Fig. 3 shows that the mCD98Ab of the present invention markedly reduces the blood antigen specific antibody titer of mouse by OVA immunization. BALB/c mouse was subcutaneously immunized with a suspension of complete Freund's adjuvant and Ovalbumin, and the serum was collected 10 days later. With the first day of immunity immunization of the mouse as 0, control antibody or anti-CD98 antibody, each 200 µg, was intraperitoneally administered to the mouse on day 2, day 4, day 6 and day 8. Ovalbumin was solidified at a concentration of 50 µg/ml in a 96 well plate, and reacted with the collected serum. Ovalbumin antibody that reacted with Ovalbumin was detected with HRP-labeled anti-mouse IgG antibody. This Figure shows OD value measured after addition of a substrate.

Fig. 4 shows that the anti-mouse CD98 antibody (3-142 antibody) of the present invention eliminates marginal zone B cells in mouse spleen cells. BALB/c mouse was intravenously administered once with rat IgG (200 µg) as a control, or with mCD98Ab (200 µg). One day later, the spleen was removed and stained with anti-CD19 antibody, anti-CD23 antibody, and anti-CD21 antibody. The Figures show expression patterns of CD23 and CD21 in CD19 positive cells. In mCD98Ab administration group, the marginal zone B cells of CD21^{high}CD23^{low} have almost disappeared as compared to the control.

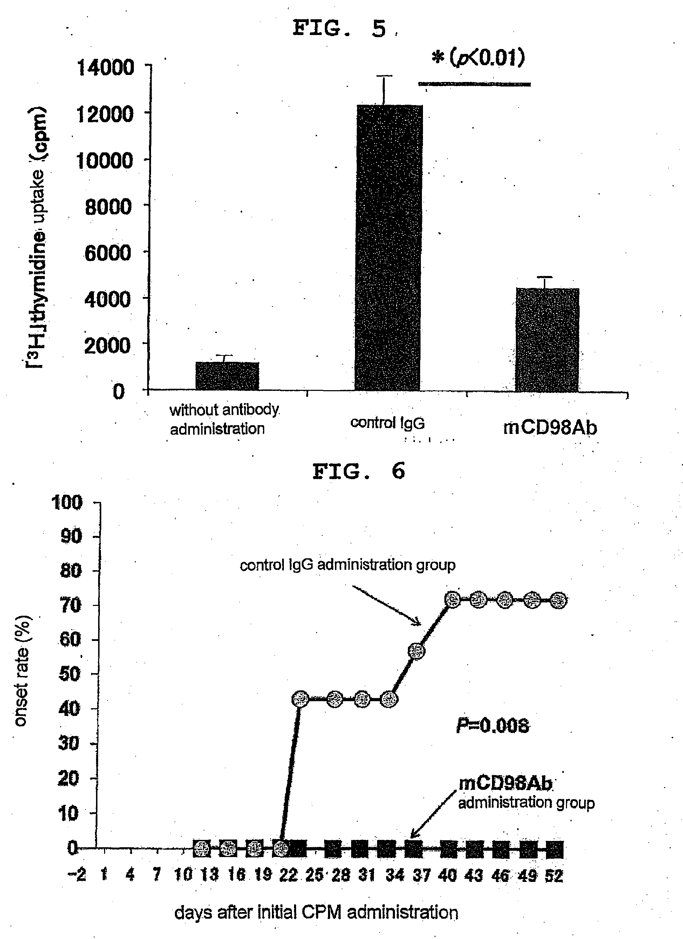

Fig. 5 shows that the mCD98Ab of the present invention shows a suppressive effect on T lymphocyte activation. To evaluate the function of the mCD98Ab of the present invention, the following T lymphocyte stimulation system was used for the study. First, spleen cells of C57BL/6 mouse and BALB/c mouse were separated, and hemolyzed using ammonium chloride solution. Then, spleen cells of BALB/c mouse were cultured in 50 µg/ml mitomycin C solution for 45 min, and DNA synthesis was ceased. Then, spleen cells of C57BL/6 mouse and spleen cells of BALB/c mouse were mix-cultured for 3 days. [³H]-thymidine was added 6 hr before completion of the culture, and the uptake thereof was verified after the completion of the culture by liquid scintillation counter.

Fig. 6 shows that the mCD98Ab of the present invention dramatically suppressed the onset in a model mouse of type 1 diabetes, wherein ■ shows an anti-CD98 antibody administration group and ● shows a control group.

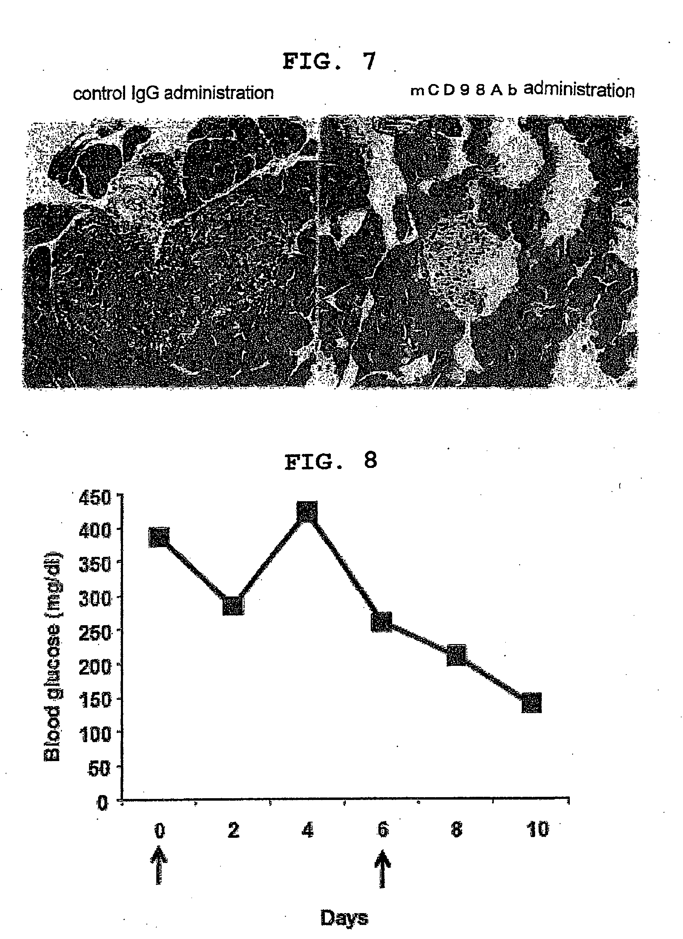

Fig. 7 shows photographs for comparison of pancreas tissue specimens of type 1 diabetes-model mouse administered with the mCD98Ab of the present invention and control type 1 diabetes-model mouse. β cell was not destroyed in the pancreas of the mCD98Ab administration group, and cell infiltration was remarkably suppressed as compared to the pancreas of the control group.

Fig. 8 shows that administration of anti-mouse CD98 antibody to a diabetes model mouse after the onset of type 1 diabetes remarkably improves blood glucose level. It is shown that administration of the anti-CD98 antibody of the present invention enables treatment of type 1 diabetes.

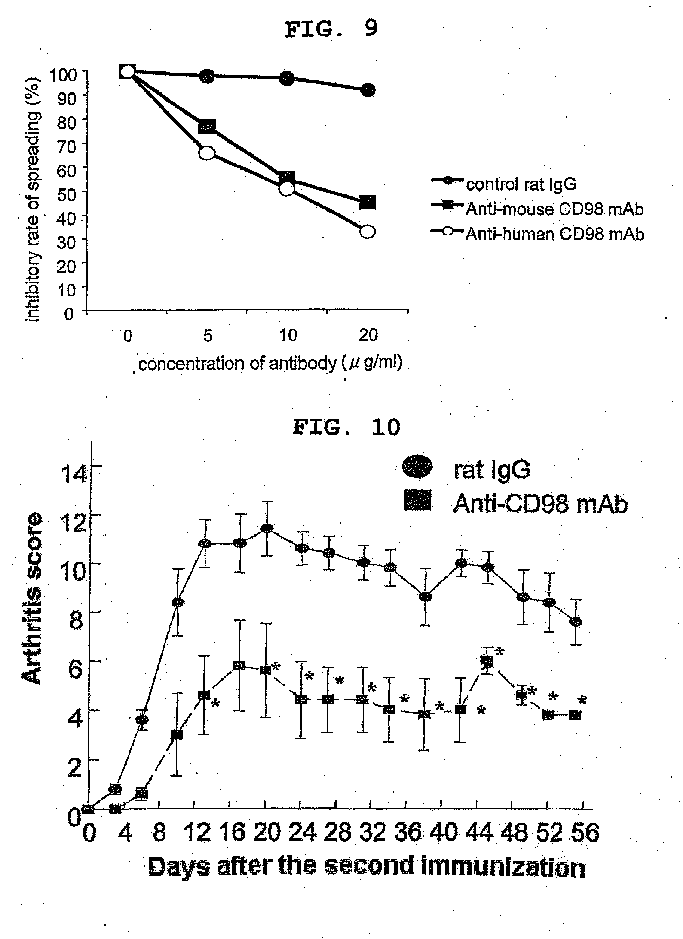

Fig. 9 shows that the anti-human CD98 antibody and anti-mouse CD98 antibody of the present invention exhibit an action to inhibit cell adhesion mediated by fibronectin. It is shown that the cell adhesion inhibitory effect is improved depending on the concentration of the antibody.

Fig. 10 shows that the onset and progression of arthritis in a collagen-induced arthritis-model mouse can be suppressed by the administration of anti-mouse CD98 antibody before onset of arthritis.

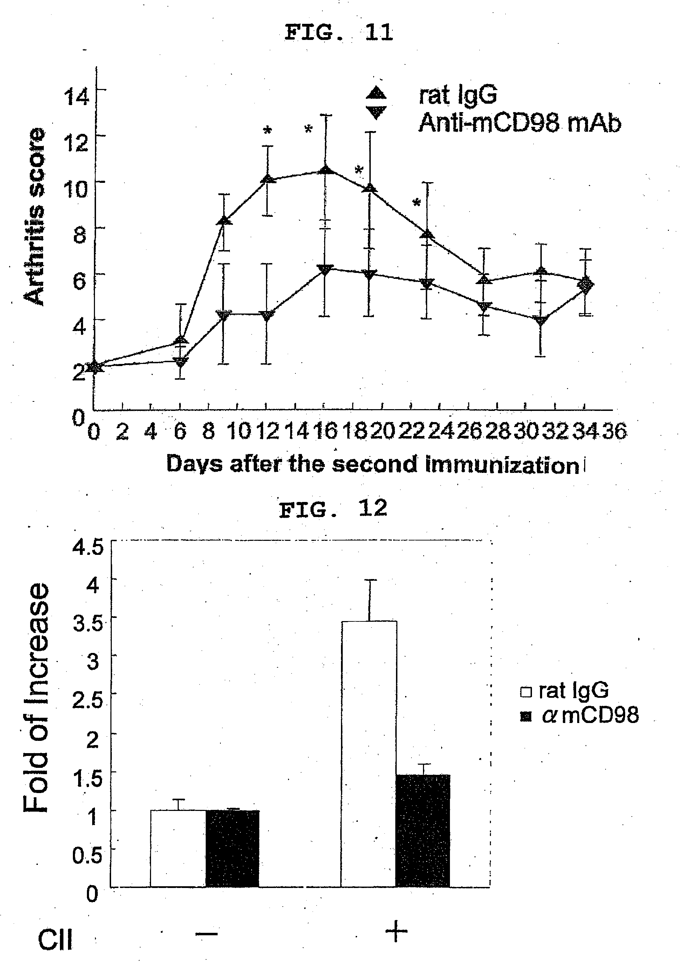

Fig. 11 shows difference in of the progression of arthritis in mice that developed collagen-induced arthritis between an anti-mouse CD98 antibody administration group and a non-administration group (rat IgG administration group). It is shown that the anti-CD98 antibody of the present invention markedly suppresses the progression of arthritis even in already developed arthritis.

Fig. 12 shows increase or decrease of [3H]-thymidine uptake rate of spleen cells isolated from mice with collagen-induced arthritis (rat IgG administration group or anti-mouse CD98 antibody administration group) and after treatment with collagen II. With the [³H]-thymidine uptake rate of collagen II untreated spleen cells as 1, increase rates based thereon are indicated.

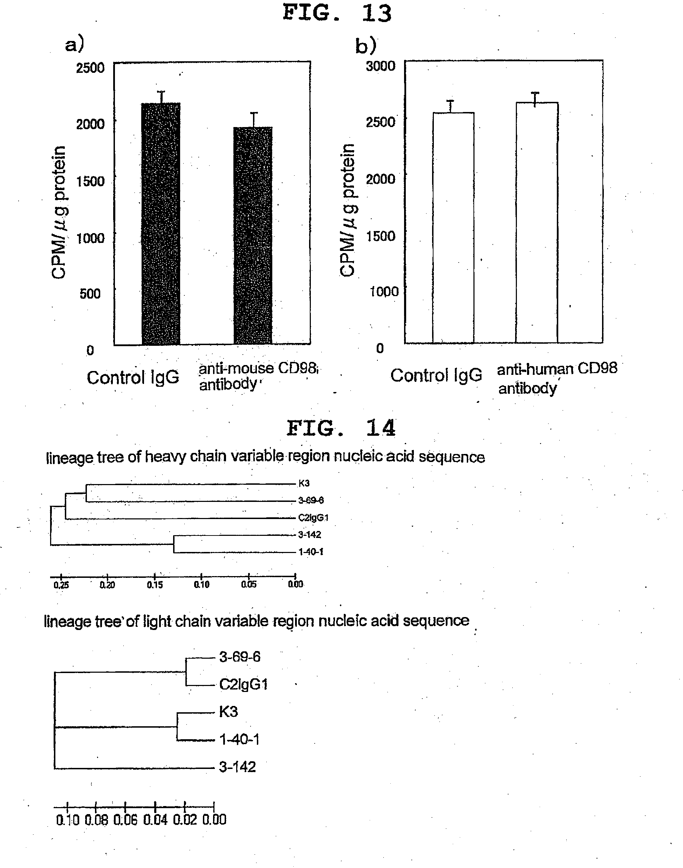

Fig. 13 shows the evaluation of the influence of anti-mouse CD98 antibody and anti-human CD98 antibody on ³H-leucine uptake of the cell, wherein a) shows the results of the evaluation of leucine uptake inhibitory effect of anti-mouse CD98 antibody by using NIH3T3 cell as the cell, and b) shows the results of the evaluation of leucine uptake inhibitory effect of anti-human CD98 antibody by using 293T cell as the cell. Both anti-CD98 antibodies did not inhibit leucine uptake of the cell.

Fig. 14 shows the lineages of variable region nucleic acid sequences relating to the anti-mouse CD98 antibody (3-142 antibody) of the present invention and conventional anti-CD98 antibodies (4 kinds of antibodies K₃, 1-40-1, 3-69-6, C2IgG1 of patent document 3).

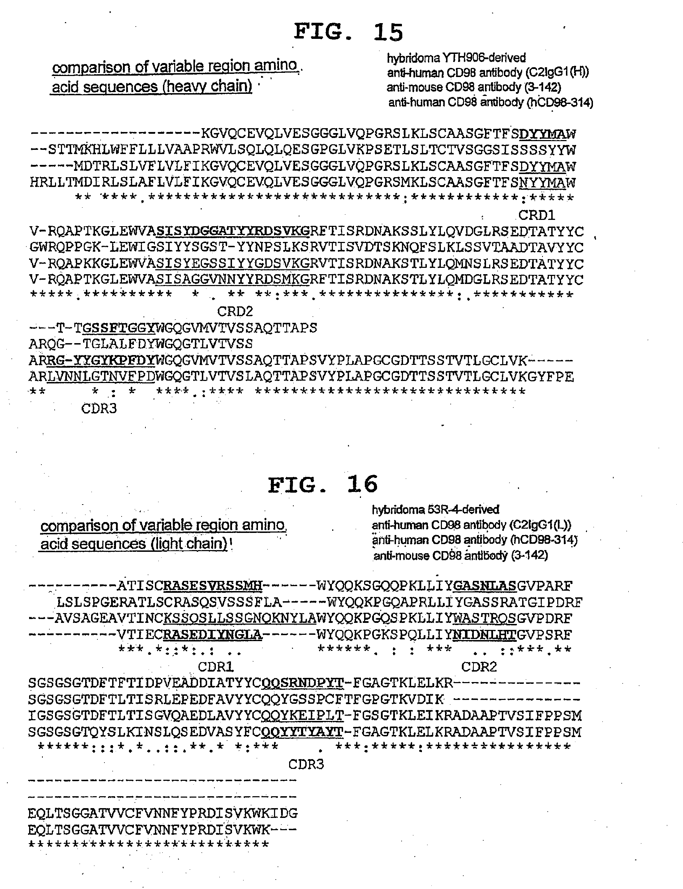

Fig. 15 is a schematic diagram showing the results of comparison of variable region amino acid sequences (heavy chain) between the anti-CD98 antibody of the present invention and conventional anti-CD98 antibody. The positions of CDR1 - 3 in the heavy chain were determined in reference to the sequence of variable region (Rat (hybridoma YTH906) immunoglobulin variable region (http://www.ncbi.nlm.nih.gov/nuccore/M87783.1)) of existing antibody. In addition, the sequence of C2IgG1 heavy chain described in patent document 3, which is a conventional anti-CD98 antibody, was referred to.

Fig. 16 is a schematic diagram showing the results of comparison of variable region amino acid sequences (light chain) between the anti-CD98 antibody of the present invention and conventional anti-CD98 antibody. The positions of CDR1 - 3 in the light chain were determined in reference to the sequence of variable region (Rattus norvegicus (hybridoma 53R-4) immunoglobulin rearranged kappa-chain mRNA variable (V) region, partial ds (http://www.ncbi.nlm.nih.gov/nuccore/L07407.1)) of existing antibody. In addition, the sequence of C2IgG1 light chain described in patent document 3, which is a conventional anti-CD98 antibody, was referred to.

### Description of Embodiments

The term "antibody" in the present invention is used in the broadest sense and includes any antibody such as monoclonal antibody (including full-length monoclonal antibody), polyclonal antibody, antibody variant, smaller antibody, multi-specific antibody (e.g., bispecific antibody), chimeric antibody, humanized antibody, human antibody and the like, as long as it exhibits a desired biological activity (activity as the below-mentioned anti-CD98 antibody).

The "anti-CD98 antibody" of the present invention is an antibody having a specific binding ability to CD98 of a disulfide bond form of CD98 heavy chain and CD98 light chain (having an amino acid transport activity). In one embodiment, the anti-CD98 antibody of the present invention has 2 sequences of SEQ ID NOs: 2 and 4 as a heavy chain variable region and a light chain variable region. In another embodiment, the anti-CD98 antibody of the present invention has 2 sequences of SEQ ID NOs: 6 and 8 as a heavy chain variable region and a light chain variable region.

For example, the anti-CD98 antibody of the present invention has the amino acid sequence shown by SEQ ID NO: 13 as heavy chain CDR1, the amino acid sequence shown by SEQ ID NO: 14 as heavy chain CDR2, and the amino acid sequence shown by SEQ ID NO: 15 as heavy chain CDR3, and the amino acid sequence shown by SEQ ID NO: 16 as light chain CDR1, the amino acid sequence shown by SEQ ID NO: 17 as light chain CDR2, and the amino acid sequence shown by SEQ ID NO: 18 as light chain CDR3.

For example, the anti-CD98 antibody of the present invention has the amino acid sequence shown by SEQ ID NO: 19 as heavy chain CDR1, the amino acid sequence shown by SEQ ID NO: 20 as heavy chain CDR2, and the amino acid sequence shown by SEQ ID NO: 21 as heavy chain CDR3, and the amino acid sequence shown by SEQ ID NO: 22 as light chain CDR1, the amino acid sequence shown by SEQ ID NO: 23 as light chain CDR2, and the amino acid sequence shown by SEQ ID NO: 24 as light chain CDR3.

The anti-CD98 antibody of the present invention characteristically a) has at least a binding site in the heavy chain of CD98, b) shows no influence on amino acid uptake by CD98, and c) suppresses activation of T lymphocyte (T cell). Preferably, it characteristically d) suppresses cell adhesion mediated by fibronectin, in addition to the above-mentioned characteristics a) - c).

Since the anti-CD98 antibody of the present invention suppresses activation of T lymphocyte, it consequently suppresses the production of OVA antibody in an OVA immunization model. Accordingly, a more preferable embodiment of the anti-CD98 antibody of the present invention provides a characteristic in that e) the production of OVA antibody is suppressed in an OVA immunization model, in addition to the above-mentioned characteristics a) - d). A particularly preferable embodiment of the anti-CD98 antibody of the present invention has the following characteristics a) - e);
a) it has a binding site in the heavy chain of CD98,
b) it does not influence amino acid uptake by CD98,
c) it has a suppressive action on T lymphocyte activation,
d) it suppresses cell adhesion mediated by fibronectin, and
e) it suppresses the production of OVA antibody in an OVA immunization model.

Preferred as the anti-CD98 antibody of the present invention includes a mouse type (anti-mouse CD98 antibody) and human type (anti-human CD98 antibody) anti-CD98 antibodies.

The anti-CD98 antibody of the present invention can be produced by a known method according to patent document 2 and the like. For example, an anti-human CD98 antibody (mouse monoclonal antibody) is produced by immunizing a non-human mammal such as mouse and the like with a human CD98 expression cell line (including both naturally expressing cell and transformed cell) (Haynes B. F et al., J. Immunol., (1981), 126, 1409-1414, Masuko T. et al., Cancer Res., (1986), 46, 1478-1484, and Freidman AW. et al., Cell. Immunol., (1994), 154, 253-263).

The anti-mouse CD98 antibody of the present invention can be obtained from an anti-mouse CD98 antibody producing cell (NITE BP-964) obtained by a known method such as the above-mentioned method and the like. Similarly, the anti-human CD98 antibody of the present invention can be obtained, for example, from an anti-human CD98 antibody producing cell (NITE BP-1007).

The functional antigen-binding fragment of the anti-CD98 antibody according to the present invention refers to a fragment of the anti-CD98 antibody specifically binding to the antigen to which the anti-CD98 antibody specifically binds, and more specifically includes heavy chain and light chain variable region (V_{H} and V_{L}), F(ab')₂, Fab', Fab, Fv, Fd, disulphide-linked FV(sdFv), Single-Chain FV(scFV) and polymers thereof, and the like (D. J. King., Applications and Engineering of Monoclonal Antibodies., 1998 T. J. International Ltd). These antibody fragments can be obtained by a conventional method, for example, digestion of an antibody molecule by a protease such as papain, pepsin and the like, or by a known genetic engineering technique. Alternatively, a gene encoding such antibody fragment is constructed, introduced into an expression vector, and expressed in a suitable host cell (see, for examples, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

The functional antigen-binding fragment of the antibody of the present invention preferably has a smaller molecular weight than the full-length antibody. For example, it may form a polymer such as dimer, trimer, tetramer and the like, and sometimes has a larger molecular weight than the full-length antibody.

The anti-CD98 antibody of the present disclosure also encompasses a monoclonal antibody consisting of a heavy chain and/or a light chain constituting the antibody, which have amino acid sequences wherein one or several amino acids is/are deleted, substituted or added. Such partial alteration (deletion, substitution, insertion, addition) of the amino acids can be introduced into the amino acid sequence of the anti-CD98 antibody of the present invention by partially altering the base sequence encoding the amino acid sequence. Such partial alteration of the base sequence can be introduced by a conventional method and using known site-directed mutagenesis (Site specific mutagenesis) (Proc Natl Acsd Sci USA., 1984 Vol. 81 5662-5666; Sambrook et al., Molecular Cloning A Laboratory Manual(1989) Second edition, Cold Spring Harbor Laboratory Press).

The anti-CD98 antibody according to the present invention includes an antibody having any immunoglobulin class and subclass. In a preferred embodiment of the present invention, the antibody is an antibody of human immunoglobulin class and subclass, and preferred class and subclasses are immunoglobulin G (IgG).

The anti-CD98 antibody of the present invention can be produced by, for example, the method described below.

CD98 or a part thereof, or a product bonded to a substance suitable for enhancing antigenicity of antigen (e.g., Keyhole limpet hemocyanin and bovine serum albumin etc.), or a cell expressing a large amount of CD98 on cell surface, is used together with a immunostimulator (Freund's adjuvant etc.) as necessary to immunize a non-human mammal such as rat, mouse, rabbit, goat, horse and the like, or immunize a non-human mammal by administration of an expression vector incorporating CD98. The CD98 antibody of the present invention can be obtained by preparing a hybridoma from an antibody producing cell obtained from the immunized animal and a myeloma cell without autoantibody producibility, cloning the hybridoma, and selecting a clone that produces a monoclonal antibody showing specific affinity for the antigen used for immunization.

The production method of the antibody of the present invention is further specifically explained below. However, the production method is not limited thereto, and for example, an antibody producing cell other than spleen cell and myeloma can also be used.

As the antigen, CD98 or a part thereof, and a cell naturally expressing CD98, as well as a transformed strain itself, which is obtained by incorporating a DNA encoding CD98 into an expression vector for animal cells, and introducing the expression vector into an animal cell, can be used.

As the expression vector for animal cells, for example, vectors such as pEGF-N1 (manufactured by Becton Dickinson Bioscience Clontech) can be used. A vector for introducing an intended gene can be prepared by cleaving an insertion site by an appropriate restriction enzyme and linking the DNA encoding CD98 cleaved by the same enzyme. The prepared expression vector can be introduced into cells, for example, L929 cells (American Type Culture Collection No. CCL-1).

The methods for introducing a gene into a host are known and include, for example, any methods (for example, a method using a calcium ion, an electroporation method, a spheroplast method, a lithium acetate method, a calcium phosphate method, a lipofection method and the like

The thus-produced transformed cell can be used as an immunogen for the production of a CD98 antibody. Furthermore, the expression vector itself can also be used as an immunogen.

CD98 can be produced by appropriately using, based on known base sequences or amino acid sequences, gene recombination techniques, as well as methods known in the technical field such as chemical synthesis methods, cell culture methods and the like. An anti-CD98 antibody can also be produced using the thus-obtained CD98 protein as an antigen. The partial sequence of CD98 can also be produced by a gene recombination technique or chemical synthesis method according to the below-mentioned method known in the technical field, and can be produced by appropriately cleaving CD98 with protease and the like.

The antigen obtained as described above can be used for immunization as described below. Specifically, the prepared antigen is mixed with an appropriate substance for enhancing the antigenicity (e.g., Keyhole limpet hemocyanin, bovine serum albumin etc.) and an immunostimulant (Freund's complete or incomplete adjuvant etc.), as required, and used for immunization of a non-human mammal such as a mouse, rabbit, goat, and a horse. The animal to be immunized receives immunization plural times at 3 - 10 day intervals. For example, when a cell is used as an antigen, the number of cells to be used for a single immunization may be any. Generally, 10³ - 10⁸ cells, for example, 10⁶ cells are used for immunization. When protein or peptide is used as an antigen, 1 - 100 µg is generally used for immunization. Immunocompetent cells are collected from an animal that underwent multiple immunizations, and the cells that produce the object antibody are cloned to give the monoclonal antibody of the present invention. The immunocompetent cell means a cell having an antibody-producing ability in an immunized animal.

A hybridoma secreting a monoclonal antibody can be prepared by a method of Köhler and Milstein (Nature., 1975 Vol.256:495-497) or in accordance with the method. In other words, a hybridoma is prepared by cell fusion between antibody producing cells contained in the spleen, lymph nodes, bone marrow, tonsil, or the like, preferably contained in the lymph nodes or spleen, obtained from an animal immunized as described above, and myeloma cells that are derived preferably from a mammal such as a mouse, a rat, a guinea pig, hamster, a rabbit, a human or the like and incapable of producing any autoantibody. Cell fusion can be performed by mixing antibody-producing cells with myeloma cells in a high concentration solution of a polymer such as polyethylene glycol (for example, molecular weight of 1500 to 6000) usually at about 30 - 40°C, for about 1 - 10min. A hybridoma clone producing a monoclonal antibody can be screened by culturing a hybridoma on, for example, a microtiter plate, and measuring reactivity of a culture supernatant from wells in which the hybridoma is grown to an immunization antigen using an immunological method such as an enzyme immunoassay (for example, ELISA), a radioimmunoassay, or a fluorescent antibody method.

A monoclonal antibody can be produced from a hybridoma by culturing the hybridoma in vitro and then isolating monoclonal antibodies from a culture supernatant. A monoclonal antibody can also be produced by a hybridoma by culturing the hybridoma in ascites or the like of a mouse, a rat, a guinea pig, a hamster, a rabbit, or the like in vivo and isolating the monoclonal antibody from the ascites. Further, a recombinant antibody can be prepared by a genetic recombination technique by cloning a gene encoding a monoclonal antibody from antibody-producing cells such as a hybridoma and the like, and incorporating the gene into an appropriate vector, introducing the vector to a host (for example, cells from a mammalian cell line, such as Chinese hamster ovary (CHO) cells and the like, E. coli, yeast cells, insect cells, plant cells and others (P. J. Delves. ANTIBODY PRODUCTION ESSENTIAL TECHNIQUES., 1997, WILEY, P. Shepherd and C. Dean., Monoclonal Antibodies., 2000, OXFORD UNIVERSITY PRESS , J. W. Goding, Monoclonal Antibodies: principles and practice., 1993, ACADEMIC PRESS). Further, by preparing a transgenic bovine, goat, sheep or porcine in which a gene of a target antibody is incorporated into an endogenous gene using a transgenic animal production technique, a large amount of a monoclonal antibody derived from the antibody gene may be obtained from the milk of the transgenic animal. When a hybridoma is cultured in vitro, the hybridoma is grown, maintained, and stored depending on the various conditions such as properties of a cell species to be cultured, objectives of a study, and culture methods and the like, and the culture may be conducted using a known nutritional medium or any nutritional mediums induced and prepared from a known basic medium that are used to produce a monoclonal antibody in a culture supernatant.

The produced monoclonal antibody can be purified by a method known in the art, for example, by appropriate combination of chromatography using a protein A column, ionexchange chromatography, hydrophobic chromatography, ammonium sulfate salting-out, gel filtration, affinity chromatography, and the like.

Furthermore, the anti-CD98 antibody of the present invention can also be produced by genetic engineering based on the base sequence information of the variable region. For example, an expression vector comprising the two sequences of SEQ ID NO: 1 and 3, or SEQ ID NOs: 5 and 7 is introduced into a host, the host is cultured, and the antibody can be obtained from the culture. SEQ ID NOs: 1 and 3 may be introduced into the same expression vector, or may be introduced into different expression vectors. Similarly, SEQ ID NOs: 5 and 7 may be introduced into the same expression vector, or may be introduced into different expression vectors. As the expression vector and host, those similar to the above-mentioned are used.

Using an expression vector constructed to express said anti-CD98 antibody, a cell expressing the anti-CD98 antibody of the present invention or a functional fragment thereof can be prepared. To be specific, a DNA encoding the object antibody is incorporated into an expression vector. At that time, the DNA is incorporated into the expression vector such that expression occurs under control of an expression regulatory region such as enhancer and promoter. Then, the host cell transformed with the expression vector expresses the antibody. In this case, a suitable host and an expression vector can be used in combination.

Examples of the vector include M13-type vector, pUC-type vector, pBR322, pBluescript, pCR-Script and the like. When subcloning of cDNA or cleaving is desired, for example, pGEM-T, pDIRECT, pT7 and the like can be used besides the above-mentioned vectors.

When a vector is used for the purpose of producing an antibody, an expression vector is particularly useful. When the host is Escherichia coli such as JM109, DH5α, HB101, XL1-Blue and the like, it is essential that the expression vector have a promoter that can be efficiently expressed in Escherichia coli, for example, lacZ promoter (Ward et al., Nature (1989) 341, 544-546; FASEB J. (1992) 6, 2422-2427, incorporated in full in the present specification by reference), araB promoter (Better et al., Science (1988) 240, 1041-1043, incorporated in full in the present specification by reference), T7 promoter and the like. Examples of such vector include pGEX-5X-1 (manufactured by Pharmacia), "QIAexpress system" (manufactured by QIAGEN), pEGFP, pET (in this case, the host is preferably BL21 expressing T7 RNA polymerase) and the like, besides the above-mentioned vectors.

In addition, the vector may contain a signal sequence for polypeptide secretion. As the signal sequence for polypeptide secretion, pelB signal sequence (Lei, S. P. et al J. Bacteriol. (1987) 169, 4397, incorporated in full in the present specification by reference) may be used when produced in the periplasm of Escherichia coli. A vector can be introduced into the host cell by, for example, a calcium chloride method or an electroporation method.

In addition, a mammal-derived expression vector (e.g., pcDNA3 (manufactured by Invitrogen), pEGF-BOS (Nucleic Acids. Res. 1990, 18(17), p5322, incorporated in full in the present specification by reference), pEF, pCDM8), an insect cell-derived expression vector (for example, "Bac-to-BAC baculovirus expression system" (manufactured by GIBCO BRL), pBacPAK8), a plant-derived expression vector (for example, pMH1, pMH2), an animal virus-derived expression vector (e.g., pHSV, pMV, pAdexLcw), a retrovirus-derived expression vector (e.g., pZIPneo), a yeast-derived expression vector (e.g., "Pichia Expression Kit" (manufactured by Invitrogen), pNV11, SP-Q01), a Bacillus subtilis-derived expression vector (e.g., pPL608, pKTH50) and the like can be used.

For expression in an animal cell such as CHO cell, COS cell, NIH3T3 cell and the like, it is essential that the vector contain a promoter necessary for intracellular expression, for example, SV40 promoter (Mulligan et al., Nature (1979) 277, 108, incorporated in full in the present specification by reference), MMTV-LTR promoter, EF1α promoter (Mizushima et al., Nucleic Acids Res. (1990) 18, 5322, incorporated in full in the present specification by reference), CAG promoter (Gene. (1991) 108, 193, incorporated in full in the present specification by reference), CMV promoter and the like, and more preferably contain a gene for selection of a transformed cell (e.g., drug resistance gene distinguishable by a drug (neomycin, G418 and the like). Examples of the vector having such property include pMAM, pDR2, pBK-RSV, pBK-CMV, pOPRSV, pOP13 and the like.

When stable expression of gene and amplification of intracellular gene copy number are desired, a method including introducing, into a CHO cell deficient in a nucleic acid synthetic pathway, a vector having a DHFR gene complementary thereto (e.g., pCHOI and the like) and amplifying same by methotrexate (MTX) can be mentioned. When a transient gene expression is desired, a method including transforming a COS cell having a gene expressing SV40 T antigen on the chromosome with a vector having an SV40 replication origin (pcD and the like) can be mentioned. As a replication origin, those derived from polioma virus, adenovirus, bovine papilloma virus (BPV) and the like can also be used. For amplification of gene copy number in a host cell system, the expression vector can contain, as a selection marker, aminoglycoside transferase (APH) gene, thymidine kinase (TK) gene, Escherichia coli xanthine guanine phosphoribosyl transferase (Ecogpt) gene, dihydrofolate reductase (dhfr) gene and the like.

The present disclosure also provides an antibody that binds to the same epitope as the epitope to which the antibody disclosed in the present invention (the above-mentioned anti-CD98 antibody) binds and use thereof as a therapeutic agent for an autoimmune disease. Said epitope can be predicted by a bioinformatics method such as sequence data analysis and steric structure analysis. In addition, whether or not the antibody binds to the same epitope can be judged by confirming the presence or absence of the competitive relationship of the both antibodies to the epitope.

The antibody used in the present disclosure may be a conjugate antibody bound to, for example, various molecules such as a nonpeptidic polymer such as polyethylene glycol (PEG) and the like, a radioactive substance, toxin and the like. Such conjugate antibody can be obtained by chemically modifying the obtained antibody. A chemical modification method has already been established in this field. The antibody in the present invention also encompasses such conjugate antibodies (D.J. King., Applications and Engineering of Monoclonal antibodies., 1998 T.J. International Ltd, Monoclonal Antibody-Based Therapy of Cancer., 1998 Marcel Dekker Inc; Chari et al., Cancer Res., 1992 Vol 152: 127; Liu et al., Proc Natl Acad Sci USA., 1996 Vol 93: 8681, all incorporated in full in the present specification by reference).

The "autoimmune diseases" in the present invention is a disease developed by the production of an autoantibody to an endogenous antigen by the immune system, for example, type 1 diabetes, rheumatoid arthritis, ulcerative colitis, psoriasis, Crohn's disease, systemic lupus erythematosus, Basedow's disease and the like. The studies made so far have clarified that these autoimmune diseases are caused by abnormal excessive activation of T cells. Particularly, it is a widely-known fact that CD4 positive or CD8 positive T lymphocytes (T cells) are excessively activated to promote autoantibody production or induce tissue destruction, which markedly affects the progression of the disease state. Since the anti-CD98 antibody of the present invention suppresses activation of T lymphocytes (T cells), the above-mentioned autoimmune diseases can be treated using the anti-CD98 antibody of the present invention. For example, as shown in Fig. 6 - Fig. 8, the anti-CD98 antibody of the present invention is effective for the treatment of type 1 diabetes and, as shown in Fig. 10 - Fig. 12, it is effective for the treatment of rheumatoid arthritis. Thus, the anti-CD98 antibody of the present invention can be used for the treatment of autoimmune diseases, for example, type 1 diabetes, rheumatoid arthritis, ulcerative colitis, psoriasis, Crohn's disease, systemic lupus erythematosus, Basedow's disease and the like. As mentioned above, preferable use of the anti-CD98 antibody of the present invention is the treatment of type 1 diabetes, rheumatoid arthritis, ulcerative colitis, psoriasis, Crohn's disease, systemic lupus erythematosus, and Basedow's disease, and particularly preferable use is the treatment of type 1 diabetes or rheumatoid arthritis.

In the present invention, the "rheumatoid arthritis" refers to an autoimmune disease with pain in the articular and bone, muscle, ligament, tendon and the like, which is an autoimmune disease developed the most frequently, shows extremely chronic progression and is difficult to treat. The disease state of rheumatoid arthritis is known to advance because CD4 positive T cells are excessively activated to produce various cytokines, as well as CD8 positive T cells destroy articular tissues. Therefore, the anti-CD98 antibody of the present invention having a suppressive action on T cell (T lymphocyte) activation is preferable for the treatment of rheumatoid arthritis.

In the present invention, "type 1 diabetes" refers to a disease where pancreatic β cells are destroyed mainly by autoimmunity, which leads to depleted insulin production and insulin shortage. When diabetes is aggravated, insulin resistance is promoted, pancreatic B cells get tired, and apoptosis starts to occur, the type 2 diabetes advances to type 1 diabetes. Both CD4 positive and CD8 positive T cells contribute to the onset of type 1 diabetes, and insulin insufficiency is finally induced by destruction of pancreatic B cells by CD8 positive T cells. Therefore, the anti-CD98 antibody of the present invention having a suppressive action on T cell (T lymphocyte) activation is preferable for the prophylaxis and treatment of type 1 diabetes.

A therapeutic agent for an autoimmune disease, containing the anti-CD98 antibody of the present invention is preferably provided as a pharmaceutical composition. Such pharmaceutical composition contains a therapeutically effective amount of the antibody, and formulated into various forms for parenteral administration. Here, the therapeutically effective amount refers to an amount affording a treatment effect for a given symptom or administration plan.

The pharmaceutical composition of the present invention may contain a pharmaceutically acceptable carrier in addition to the antibody. Examples of the pharmaceutically acceptable carrier include sterilized water and saline, stabilizer, excipient, antioxidant, buffering agent, preservative, surfactant, chelating agent, binder and the like. One or more kinds of these carriers can be contained.

As the surfactant, nonionic surfactants can be mentioned, and, for example, those having HLB 6 - 18, for example, sorbitan fatty acid ester such as sorbitan monocaprylate, sorbitan monolaurate, sorbitan monopalmitate and the like; glycerin fatty acid ester such as glycerin monocaprylate, glycerin monomyristate, glycerin monostearate and the like; polyglycerin fatty acid ester such as decaglycerylmonostearate, decaglyceryldistearate, decaglycerylmonolinoleate and the like; polyoxyethylene sorbitan fatty acid ester such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene tristearate sorbitan and the like; polyoxyethylenesorbit fatty acid ester such as polyoxyethylenesorbit tetrastearate, polyoxyethylenesorbit tetraoleate and the like; polyoxyethylene glycerine fatty acid ester such as polyoxyethyleneglycerylmonostearate and the like; polyethylene glycol fatty acid ester such as polyethylene glycol distearate and the like; polyoxyethylene alkyl ether such as polyoxyethylenelauryl ether and the like; polyoxyethylenepolyoxypropylenealkylether such as polyoxyethylenepolyoxypropyleneglycol, polyoxyethylenepolyoxypropylenepropylether, polyoxyethylenepolyoxypropylenecetyl ether and the like; polyoxyethylenealkylphenylether such as polyoxyethylenenonylphenylether and the like; polyoxyethylene hydrogenated castor oil such as polyoxyethylenecastor oil, polyoxyethylene hydrogenated castor oil (polyoxyethylene hydrogen castor oil) and the like; polyoxyethylene beeswax derivative such as polyoxyethylenesorbit beeswax and the like; polyoxyethylenelanolin derivative such as polyoxyethylenelanolin and the like; polyoxyethylene fatty acid amide such as polyoxyethylene stearic acid amide and the like and the like, and the like can be mentioned as typical examples.

In addition, as the surfactant, anion surfactants can also be mentioned and, for example, alkyl sulfate having a C₁₀₋₁₈ alkyl group such as cetyl sodium sulfate, sodium lauryl sulfate, sodium oleyl sulfate and the like; polyoxyethylene alkyl ether sulfate wherein the average addition number of moles of ethylene oxide is 2 - 4 and the alkyl group has 10 - 18 carbon atoms such as polyoxyethylene sodium lauryl sulfate and the like; alkylsulfosuccinic acid ester salt wherein the alkyl group has 8 - 18 carbon atoms such as sodium lauryl sulfosuccinate and the like; natural surfactants such as lecithin, glycerolphospholipid; fingophospholipid such as sphingomyelin and the like; sucrose fatty acid ester of fatty acid having 12 - 18 carbon atoms and the like can be mentioned as typical examples.

As the buffering agent, organic acids such as phosphoric acid, citrate buffer, acetic acid, malic acid, tartaric acid, succinic acid, lactic acid, potassium phosphate, gluconic acid, caprylic acid, deoxycholic acid, salicylic acid, triethanolamine, fumaric acid and the like, carbonate buffer, Tris buffer, histidine buffer, imidazole buffer and the like can be mentioned.

The pharmaceutical composition of the present invention may contain, in addition to the antibody, other polypeptide having a low molecular weight, proteins such as serum albumin, gelatin, immunoglobulin and the like, amino acid, saccharides or hydrocarbonate such as polysaccharide, monosaccharide and the like, and sugar alcohol.

As the amino acid, basic amino acid such as arginine, lysine, histidine, ornithine and the like, and inorganic salts of these amino acids (preferably in the form of hydrochloride or phosphate, that is, phosphoric acid - amino acid) can be mentioned. When a free amino acid is used, preferable pH value is adjusted by adding a suitable physiologically acceptable buffering substance, for example, inorganic acid, particularly hydrochloric acid, phosphoric acid, sulfuric acid, acetic acid, formic acid or a salt thereof. In this case, use of phosphate particularly advantageous, since a particularly stable freeze-dried product can be obtained. It is particularly advantageous when the prepared product does not substantially contain an organic acid, for example, malic acid, tartaric acid, citric acid, succinic acid, fumaric acid and the like or corresponding anion (malic acid ion, tartaric acid ion, citric acid ion, succinic acid ion, fumaric acid ion etc.) is absent. Preferable amino acid is arginine, lysine, histidine or ornithine. Moreover, acidic amino acid, for example, glutamic acid and aspartic acid, and a salt thereof (preferably sodium salt) or neutral amino acid, for example, isoleucine, leucine, glycine, serine, threonine, valine, methionine, cysteine or alanine, or aromatic amino acid, for example, phenylalanine, tyrosine, tryptophan, or a derivative (N-acetyltryptophan) can also be used.

As the saccharides or hydrocarbonate such as polysaccharide, monosaccharide and the like, dextran, glucose, fluctose, lactose, xylose, mannose, maltose, sucrose, trehalose, raffinose and the like can be mentioned.

As the sugar alcohol, mannitol, sorbitol, inositol and the like can be mentioned.

When the pharmaceutical composition of the present invention is prepared as an aqueous solution for injection, it can be mixed with saline or an isotonic solution containing, for example, glucose and other auxiliary agent (e.g., D-sorbitol, D-mannose, D-mannitol, sodium chloride). The aqueous solution may be used in combination with a suitable solubilizing agent (for example, alcohol (ethanol etc.), polyalcohol (propylene glycol, PEG etc.), non-ionic surfactant (polysorbate 80, HCO-50) etc.). When desired, diluent, solubilizing agent, pH adjuster, soothing agent, sulfur-containing reducing agent, antioxidant and the like may be contained.

As the sulfur-containing reducing agent, N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione, and those having a sulfhydryl group such as C₁₋₇ thioalkanoic acid and the like, and the like can be mentioned.

As the antioxidant, moreover, erythorbic acid, dibutylhydroxytoluene, butylhydroxyanisole, α-tocopherol, tocopherol acetate, L-ascorbic acid and a salt thereof, L-ascorbic acid palmitate, L-ascorbic acid stearate, sodium bisulfite, sodium sulfite, triamyl gallate, propyl gallate or chelating agent such as ethylenediaminetetraacetic acid disodium (EDTA), sodium pyrophosphate, sodium metaphosphate and the like can be mentioned.

Where necessary, the composition may be sealed in a microcapsule (microcapsule of hydroxymethylcellulose, gelatin, poly[methylmethacrylic acid] and the like), or can also be prepared as a colloid drug delivery system (liposome, albumin microsphere, microemulsion, nanoparticles and nanocapsule etc.) (see "Remington's Pharmaceutical Science 16th edition", Oslo Ed., 1980 etc.). Moreover, a method of processing a medicament into a sustained-release medicament is also known, and can be applied to the present invention (Langer et al., J. Biomed. Mater. Res. 1981, 15:167-277; Langer, Chem. Tech. 1982, 12:98-105; US Patent No. 3,773,919; EP-A-58,481; Sidman et al., Biopolymers 1983, 22: 547-556; EP-B-133,988).

As the preparation form, a preparation such as freeze-dried preparation (which can be reconstituted by the addition of the above-mentioned aqueous buffering solution and used), sustained release preparation, enteric preparation, injection, drip infusion and the like can be selected according to the object of treatment and treatment plan.

While the administration route may be appropriately determined, for example, oral administration, as well as parenteral administration such as intravenous, intramuscular, subcutaneous and intraperitoneal injections and the like can be mentioned. Alternatively, directly contacting the anti-CD98 antibody of the present invention to the affected part of patients is also possible.

While the dose is appropriately determined based on tests using animals and clinical tests, generally, the conditions or severity, age, body weight, sex and the like of the patients should be taken into consideration. Generally, about 0.01 mg - 1000 mg per day can be administered to an adult by oral administration at once or in several portions. For parenteral administration, about 0.01 mg - 1000 mg per administration can be administered by subcutaneous injection, muscular injection or intravenous injection.

In a preferable embodiment of the present disclosure, the anti-CD98 antibody of the present invention is used as an ampoule containing a sterile solution or suspension of the antibody in water or other pharmacologically acceptable solution. In addition, an aseptic powder preparation (preferably lyophilized antibody of the present invention) may be filled in an ampoule, and may be diluted with a pharmacologically acceptable solution when in use.

Moreover, the anti-CD98 antibody of the present invention can also be prepared as a mixture with other medicament useful for a combined use. Specifically, as other medicament, an antibody other than the anti-CD98 antibody of the present invention, a therapeutic agent for an autoimmune disease, a therapeutic agent for diabetes and the like can be mentioned. Such medicament used in combination with the anti-CD98 antibody of the present invention is also referred to as a concomitant drug.

Examples of the antibody other than the anti-CD98 antibody of the present invention include the antibodies provided in patent documents 1 and 2.

Examples of the therapeutic agent for autoimmune diseases include a therapeutic agent for rheumatoid arthritis and the like.

Examples of the therapeutic agent for diabetes include insulin preparation, α-glucosidase inhibitor, biguanide, insulin secretagogue, dipeptidyl peptidase-IV (DPP-IV) inhibitor, GLP-1, GLP-1 analogue, protein tyrosine phosphatase inhibitor, β3 agonist and the like.

Two or more kinds of the above-mentioned concomitant drug may be used in combination at an appropriate ratio.

### Examples

The present invention is now explained in more detail in the following by referring to Examples, which are not to be construed as limitative.

### (Example 1) Preparation of anti-mouse CD98 antibody

Bone marrow cells were separated from a BALB/c mouse, and said bone marrow cells were cultured in the presence of GM-CSF for 8 days. Thereafter, 8-week-old Wistar rat was immunized with the cells (1x10⁷ cells) in the abdominal cavity. Two weeks from the first immunization, immunization was repeated. Then, 2 weeks later, the rat was immunized again, and the spleen was taken out from the rat 3 days later. Using the spleen cell and SP2 cell (myeloma cell), cell fusion was performed according to a conventional method and selected in HAT medium. Thereafter, using a flow cytometer, an antibody responsive to a dendritic cell was selected. A single clone of the selected antibody was collected using a limit dilution method. Since the limit dilution method was performed three times under the condition of 0.5 cell/well, the clone is considered to be a single clone. Not less than 40 kinds of antibodies could be isolated by the above-mentioned method.

The obtained antibody was added to a T cell culture system (mixed lymphocyte reaction obtained by mixing T cell of BALB/c mouse and spleen cell of C57BL/6 mouse) and an antibody that suppresses growth of T cell was selected. Ten kinds of such antibodies were selected. To identify a molecule recognized by one (mCD98Ab) of the antibodies, the spleen cell of BALB/c mouse was solubilized with NP40, and the antibody was added to the solubilized protein molecule mixture. The antibody·protein complex was isolated using protein G and separated using SDS gel. The separated protein was visualized by silver staining. As shown in Fig. 1, a specifically reacting 75kD band was cleaved out and identified by a mass spectrometry (LC-MAS). As a result, the recognized molecule was clarified to be a CD98 heavy chain.

Said antibody did not react by Western blotting and was found to be an antibody recognizing a higher-order structure. The anti-mouse CD98 antibody producing cell which produces the antibody was domestically deposited (NITE P-964) in the National Institute of Technology and Evaluation Patent Microorganisms Depositary (2-5-8 Kazusakamatri, Kisarazu-shi, Chiba, Japan) on July 8, 2010, transferred to the international deposit under the Budapest Treaty on February 7, 2011, and accorded with accession No. NITE BP-964 (date of original deposit: July 8, 2010). The antibody produced by NITE BP-964 is also indicated as 3-142 antibody.

### (Example 2) Reactivity of anti-mouse CD98 antibody (mCD98Ab) of the present invention

To confirm the reactivity of the mCD98Ab of the present invention, CD98-expressing CHO cell (293T/CD98) was produced by introducing mouse CD98 gene (mCD98, GenBank/EMBL/DDBJ accession no. U25708). That is, the mouse CD98 gene incorporated into pcDNA3.1 or pcDNA3.1 alone was transfected into the CHO cell by an electroporation method. To select a cell having CD98 gene incorporated into CHO cell genome, drug selection was performed using G418, and a drug resistance cell was isolated. The cell was stained with rat immunoglobulin G (control antibody) or mCD98Ab, washed to remove free antibody, and stained with PE-labeled anti-rat IgG. The results are shown in Fig. 2. The mCD98Ab of the present invention did not react with a cell without introduction of CD98 gene and reacted with a cell introduced with the CD98 gene. The left Figure shows a cell transfected with pcDNA3.1 alone, and the right Figure shows a cell introduced with the mouse CD98 gene.

The above-mentioned results reveal that the mCD98Ab of the present invention specifically recognizes mouse CD98 expressed in CHO cell.

### (Example 3) Anti-inflammatory action of anti-mouse CD98 antibody (mCD98Ab) of the present invention

To evaluate the function of mCD98Ab of the present invention, an OVA immunization model was produced. That is, a BALB/c mouse was immunosensitized by subcutaneous injection of a complex of a mixture of an OVA (ovalbumin) protein and a complete Freund's adjuvant. With the first day of immune sensitization of the mouse as day 0, a control antibody or an anti-CD98 antibody (each 200 µg) was intraperitoneally administered to the mouse on days 2, 4, 6 and 8. On day 10, the serum was separated from the mouse, and OVA-specific IgM and IgG therein were measured by the ELISA method. That is, ovalbumin was solid-phased on a 96-well plate at a concentration of 50 µg/ml, and reacted with the collected serum. An anti-Ovalbumin antibody that reacted with Ovalbumin was detected by HRP-labeled anti-mouse IgG.

The results are shown in Fig. 3. As shown in Fig. 3, a remarkably decreased production of the OVA antibody was observed in the group administered with the anti-mouse CD98 antibody of the present invention. Since an action to remarkably decrease blood antigen-specific antibody titer was not observed with already known anti-CD98 antibodies, the action was considered to be the feature of the anti-CD98 antibody of the present invention.

### (Example 4) Effect of anti-mouse CD98 antibody (mCD98Ab) of the present invention on marginal zone B cells

To evaluate the function of the mCD98Ab the present invention, an effect on marginal zone B cells was confirmed. First, a control antibody or an anti-CD98 antibody (each 200 µg) was intraperitoneally administered to a BALB/c mouse. Two days after administration, the mouse spleen cell was separated, stained with a mouse-anti CD23 antibody and a mouse-anti CD21 antibody and examined by a flow cytometer.

The results are shown in Fig. 4. Disappearance of CD21^{high}CD23^{low} fraction was observed in the anti-mouse CD98 antibody administration group. This fraction is a cell population called marginal zone B cells. Such action (disappearance of marginal zone B cells) has not been observed with known anti-CD98 antibodies.

### (Example 5) Suppressive effect on T lymphocyte activation of anti-mouse CD98 antibody (mCD98Ab) of the present invention

To evaluate the function of the mCD98Ab of the present invention, study was performed using the following T lymphocyte stimuli system. At first, the spleen cells of C57BL/6 mouse and BALB/c mouse were separated and hemolyzed using an ammonium chloride solution. Thereafter, the spleen cells of the BALB/c mouse were cultured in a 50 µg/ml mitomycinC solution for 45 min, and an operation was performed to cease a DNA synthesis. Thereafter, the spleen cells of the C57BL/6 mouse and the spleen cells of the BALB/c mouse were cultured in mixture for 3 days. [³H]-thymidine was added before 6 hr of the completion of culture, and after the completion of the culture, an uptake thereof was verified with a liquid scintillation counter. A control IgG or mCD98Ab was each added 20 µg/ml simultaneously with the addition of the [³H]-thymidine.

The results are shown in Fig. 5. The mCD98Ab of the present invention is shown to suppress a T lymphocyte stimulation activity.

### (Example 6) Treatment effect of the anti-mouse CD98 antibody of the present invention on type 1 diabetes-model mouse

To evaluate the function of mCD98Ab of the present invention, the following type 1 diabetes-model mouse was produced, the anti-mouse CD98 antibody of the present invention was administered and the effect thereof was confirmed.

The type 1 diabetes-model is a system that develops diabetes by two times (2 week interval) of intraperitoneal administration of cyclophosphamide (CPM) to NOD mouse (female, 10-week-old). When fasting blood glucose is not less than 250 mg/dl two times in a row, the mouse is diagnosed to have diabetes. NOD mouse is a long-known model mouse of type 1 diabetes and is also known to spontaneously develop diabetes. However, administration of CPM can induce diabetes in early stages. Hence, this model was used at this time.

### (1) type 1 diabetes onset preventive effect by anti-mouse CD98 antibody:

First, an anti-mouse CD98 antibody (200 µg/mouse) was administered on the same day as the first CPM administration, and intraperitoneally administered 6 times every 3 days. As a control, a rat IgG antibody (200 µg/mouse) was used instead of the anti-mouse CD98 antibody, and intraperitoneally administered 6 times every 3 days in the same manner. The results are shown in Fig. 6, wherein ■ shows an anti-mouse CD98 antibody administration group and ● shows a control group. As shown in Fig. 6, administration of the anti-mouse CD98 antibody remarkably suppressed the onset of diabetes.

Furthermore, the pancreas of each of the mice in the anti-mouse CD98 antibody administration group and control group was isolated and fixed with formalin, and paraffin segments were prepared. After cutting out thin layer segments, the segments were stained with HE. Microscopic photographs thereof are shown in Fig. 7. The pancreas of the control group showed necrosis-falling off of β cell as shown in the left figure of Fig. 7. However, the anti-mouse CD98 antibody administration group did not show a greatly change of β cell as shown in the right figure of Fig. 7.

### (2) Treatment effect of anti-mouse CD98 antibody after onset of type 1 diabetes:

NOD mice were observed for urine glucose and blood glucose from 15 weeks of age, and a mouse which showed positive urine glucose and casual blood glucose exceeding 200 mg/dl was evaluated to have developed diabetes. The mouse that developed diabetes was intraperitoneally administered with an anti-mouse CD98 antibody (200 µg) at the time of the onset and 6 days later. Blood glucose was measured every other day, and suppression of the onset of diabetes was examined. As shown in Fig. 8, the blood glucose level tended to remarkably decrease with two times of antibody administration. From this, it has been clarified that the anti-CD98 antibody of the present invention provides a treatment effect effective even for type 1 diabetes developed once.

### (Example 7) Production of anti-human CD98 antibody

The anti-human CD98 antibody of the present invention was produced as follows. First, human CD98 gene (hCD98, GenBank/EMBL/DDBJ accession no. AB018010) was introduced into a CHO cell by an electroporation method. Since a plasmid incorporating human CD98 gene contains a neomycin resistance gene, a CHO cell having neomycin resistance was selected. As a result, a CHO cell constitutively expressing human CD98 (CHO-hCD98) could be established. The above-mentioned CHO-hCD98 was administered to a Wistar rat for immune sensitization (three times at 2 week intervals). A spleen cell of the immunized rat and SP2 cell were fused according to a conventional method to produce a hybridoma. Using the hybridoma and a flow cytometer, an antibody that reacts with CHO-hCD98 but does not react with CHO cell was selected. As a result, a total of 9 antibodies considered to recognize human CD98 could be established.

One anti-human CD98 antibody of these showed a cell adhesion inhibitory activity similar to that of the anti-mouse CD98 antibody in Example 1, as shown in Example 8.

The anti-human CD98 antibody producing cells of producing said antibody was domestically deposited (NITE P-1007) at the National Institute of Technology and Evaluation Patent Microorganisms Depositary (2-5-8 Kazusakamatri, Kisarazu-shi, Chiba, Japan) on November 30, 2010, transferred to the international deposit under the Budapest Treaty on February 7, 2011, and accorded accession No. NITE BP-1007 (date of original deposit: November 30, 2010). An antibody produced by NITE BP-1007 is also indicated as hCD98-314 antibody.

### (Example 8) Test of cell adhesion inhibitory effect of anti-human CD98 antibody and anti-mouse CD98 antibody of the present invention

According to the method of Example 7, a mouse CD98 gene or human CD98 gene was introduced into a CHO cell, and a CHO cell line constitutively expressing a mouse CD98 or human CD98 was established by drug selection. The established cells were reacted with various concentrations of the anti-mouse CD98 antibody of Example 1 or the anti-human CD98 antibody of Example 7 at 4°C for 30 min, and the cells were washed. The above-mentioned cells were seeded in a 24-well plate wherein fibronectin was solid-phased, and the spreading cells were counted at 4 hr after seeding. The number of the spreading cells in the group without treatment with an antibody was taken as 100%, and the spreading inhibitory rate when treated with an antibody was evaluated.

The results are shown in Fig. 9. As shown in Fig. 9, both the anti-mouse CD98 antibody and anti-human CD98 antibody of the present invention inhibited the spreading of CHO cells. That is, the both antibodies were shown to have an equivalent ability to inhibit cell adhesion.

### (Example 9) Treatment effect of anti-mouse CD98 antibody of the present invention on rheumatoid arthritis-model mouse

An induced- arthritis model mouse was produced according to a known document (Kijima M. et al., J Immunol. 2009 Mar 15; 182 (6): 3566-72). First, an emulsion obtained by mixing and emulsifying bovine collagen II and a complete adjuvant was administered twice to DBA/1J mice (7-week-old, male, 10 mice) for immune sensitization to induce arthritis. As a result, model mice that developed arthritis with tumentia of articular and redness as main symptoms could be prepared.

### (1) Arthritis onset preventive effect of anti-mouse CD98 antibody:

Two groups of DBA/1J mice (7-week-old, male, 10 mice) were divided into an antibody administration group and a non-administration group and, 2 days prior to the administration of an emulsion obtained by mixing and emulsifying bovine collagen II and a complete adjuvant, an anti-mouse CD98 antibody (200 µg/mouse) was administered to the administration group, and rat IgG (100 µg/mouse) was administered to the non-administration group. On Days 2, 4 and 6 after the first administration of bovine collagen II, an anti-mouse CD98 antibody (200 µg/mouse) was administered to the administration group, and rat IgG (100 µg/mouse) was administered to the non-administration group, as in the above. Furthermore, on Day 26, bovine collagen II was administered for the second time, and the onset of arthritis was evaluated by observation.

The results are shown in Fig. 10. Fig. 10 provides mean±standard deviation of clinical scores. The horizontal axis shows the number of days from the second collagen immunization. As shown in the results, the anti-mouse CD98 antibody administration group showed remarkable suppression of the onset of collagen-induced arthritis as compared to the non-administration group, and the anti-mouse CD98 antibody was shown to have an arthritis preventive effect.

### (2) Treatment effect of anti-mouse CD98 antibody after onset of arthritis:

In the same manner as in the previous section, two groups of DBA/1J mice (7-week-old, male, 10 mice) were divided into an antibody administration group and a non-administration group, bovine collagen II was administered in the same manner, bovine collagen II was administered again on Day 26 and the onset of arthritis was awaited. On Days 3, 5, 7, 9 and 11 after the administration of bovine collagen II for the second time, an anti-mouse CD98 antibody (400 µg/mouse) was administered to the administration group, and rat IgG (100 µg/mouse) was administered to the non-administration group.

The results are shown in Fig. 11. In Fig. 11, the horizontal axis shows the number of days after the onset of arthritis. In addition, the clinical score is shown by mean±standard deviation. As shown in the results, the anti-mouse CD98 antibody administration group showed a good suppressive effect even for arthritis developed once, as compared to the non-administration group. Thus, the anti-mouse CD98 antibody has been shown to be effective for the treatment of arthritis.

### (Example 10) Suppressive effect of anti-mouse CD98 antibody of the present invention on the T cell activation in spleen cell of rheumatoid arthritis model mouse

After conducting the arthritis model treatment experiment in Example 9(2), bovine collagen II was administered for the second time and, on Day 20 thereafter, the spleen of each mice of the administration group and non-administration group was isolated. To the isolated mouse spleen cells was added bovine collagen II at a concentration of 50 µg/ml, and the mixture was cultured for 72 hr. Eight hours prior to the completion of culture, [³H]-thymidine (1 microcurie (µCi)) was added and, after the completion of the culture, [³H]-thymidine uptake of the spleen cells was counted by a liquid scintillation counter.

The results are shown in Fig. 12. Fig. 12 shows the increase rate with the addition of collagen, based on the group without addition of collagen (CII) as 1. From these results, administration of the anti-mouse CD98 antibody of the present invention has been found to remarkably suppress activation of T cells.

### (Example 11) Inhibitory effect of anti-mouse CD98 antibody and anti-human CD98 antibody on amino acid uptake of cell

### (1) Inhibitory effect of anti-mouse CD98 antibody:

NIH3T3 cells are treated at 37°C for 30 min with the anti-mouse CD98 antibody produced in Example 1, at a final concentration of 30 µg/ml. After treatment, the cells were washed, [³H]-leucine (1 µCi) was added, and the mixture was incubated at 37°C for 10 min. After washing the cells, lysis buffer (100 µl) was added to lyse the cells. The supernatant of the lysate was collected and the radiation dose per unit protein in the supernatant was calculated.

The results are shown in Fig. 13 a). As is clear from the Figure, the anti-mouse CD98 antibody has been shown to not at all inhibit the amino acid uptake of NIH3T3 cells, as compared to the test results obtained by adding IgG as a control.

### (2) Inhibitory effect of anti-human CD98 antibody:

Using 293T cells and the anti-human CD98 antibody produced in Example 7, the same operation as in the above-mentioned (1) was performed.

The results are shown in Fig. 13 b). As is clear from the Figure, the anti-human CD98 antibody has been shown to not at all inhibit the amino acid uptake of 293T cells, as compared to the test results obtained by adding IgG as a control.

### (Example 12) Analysis of gene encoding anti-mouse CD98 antibody or anti-human CD98 antibody

### (1) Analysis of anti-mouse CD98 antibody

The hybridoma producing the 3-142 antibody obtained in Example 1 was cultured in a RPMI1640 medium containing 10% FBS, and total RNA was extracted from about 1x10⁷ cells by using RNAiso Plus (Takara). The extraction method followed the attached document of the reagent. The total RNA was dissolved in Nuclease-free water (Ambion), and a DNA degradation treatment was performed using RNase-free DNase I (Takara) and according to the attached document. To obtain cDNA from the total RNA, a reverse transcription reaction was performed using an oligodT primer and a PrimeScript RT-PCR Kit (Takara). Using cDNA as a template and a PrimeSTAR HS DNA polymerase (Takara), the variable region of the antibody was amplified.

The primers used were 5'-CGG AAT TCA GGT (GC) (AC) A (AG) CTG CAG (GC) AGT-3' (SEQ ID NO: 9) and 5'-GCG GAT CCG GAC AGG GCT CCA GAG TTC CA-3' (SEQ ID NO: 10) for γ chain, and 5'-GCG AAT TCG ACA (CT) (CT) (AGC) (AT) G (AC) TGA C (ACT) C AGT CTC-3' (SEQ ID NO: 11) and 5'--CGC GAA GCT TTC AGT AAC ACT GT (CT) CAG GAC A-3' (SEQ ID NO: 12) for κ chain (Patrik Wernhoff et. al., Int. Immunol., 13(7) pp909-919, 2001).

The PCR for γ chain included, after heating at 98°C for 2 min, 40 cycles of three steps of 98°C for 10 sec, 57°C for 5 sec, and 72°C for 2 min. The PCR for κ chain included, after heating at 98°C for 2 min, 40 cycles of three steps of 98°C for 10 sec, 55°C for 5 sec, and 72°C for 2 min.

The PCR product was electrophoresed using a 2% agarose gel, and single bands at about 700 bp in the PCR of γ chain and about 550 bp in the PCR of κ chain were confirmed. These bands were cleaved out and purified by a QIAquick Gel Extraction Kit (Qiagen). The purified product was subjected to direct sequencing using a Big Dye Terminator v3.1 Cycle Sequencing Kit (Applied Biosystems) and the sequence was analyzed using a 3100-Avant Genetic Analyzer (Applied Biosystems).

The DNA sequence containing a heavy chain variable region and a light chain variable region and the amino acid sequence containing a heavy chain variable region and a light chain variable region of the 3-142 antibody were the sequences shown by the following SEQ ID NOs.

### <Heavy chain variable region nucleic acid sequence of 3-142 antibody>

### <Heavy chain variable region amino acid sequence of 3-142 antibody >

### <Light chain variable region nucleic acid sequence of 3-142 antibody>

### <Light chain variable region amino acid sequence of 3-142 antibody >

### (2) Analysis of anti-human CD98 antibody

In the same manner as in the analysis of the anti-mouse CD98 antibody of the above-mentioned (1), the DNA sequence containing a heavy chain variable region and a light chain variable region and the amino acid sequence containing a heavy chain variable region and a light chain variable region of the hCD98-314 antibody obtained in Example 7 were examined. The sequences are shown by the following SEQ ID NOs.

### <Heavy chain variable region nucleic acid sequence of hCD98-314 antibody>

### <Heavy chain variable region amino acid sequence of hCD98-314 antibody>

### <Light chain variable region nucleic acid sequence of hCD98-314 antibody>

### <Light chain variable region amino acid sequence of hCD98-314 antibody>

### (3) Preparation of lineage tree of anti-CD98 antibody

The DNA sequences in the variable regions of the anti-CD98 antibody of the present invention and a conventional anti-CD98 antibody were compared and a lineage tree was prepared. As the DNA sequences in the variable region of the conventional anti-CD98 antibody, those of K₃, 3-69-6, C2IgG1 and 1-40-1 reported in patent reference 3 were used.
CLUSTALW version 1.83 (http://clustalw.ddbj.nig.ac.jp/top-j.html) was activated, and the heavy chain and light chain variable region nucleic acid sequences of the 3-142 antibody obtained in the above-mentioned (1), and heavy chain and light chain variable region nucleic acid sequences of K₃, 1-40-1, 3-69-6 and C2IgG1 were respectively entered to give analysis results. Then, MEGA4.0.2 (http://www.megasoftware.net/mega4/mega.html) was activated, the data obtained in CLUSTALW were opened, and a lineage tree was formed. The results are shown in Fig. 14.

### (4) Determination of complementary region of anti-CD98 antibody

Based on the sequence information obtained in the above-mentioned (1) - (3), complementarity regions (CDR) 1 - 3 of the anti-CD98 antibody of the present invention were determined. To be specific, they were determined by comparison with the heavy chain variable region amino acid sequence or light chain variable region amino acid sequence of the conventional anti-CD98 antibody. The positions of the CDRs 1 - 3 of the heavy chain were determined by reference to the sequence (SEQ ID NO: 25) of the variable region (Rat (hybridoma YTH906) immunoglobulin variable region (http://www.ncbi.nlm.nih.gov/nuccore/M87783.1)) of the conventional antibody. In addition, they were determined by using the C2IgG1 heavy chain described in patent document 3 (SEQ ID NO: 43 in patent document 3; SEQ ID NO: 26 in the present description), which is the conventional anti-CD98 antibody, and utilizing ABG: Directory of 3D structures of antibodies (http://www.ibt.unam.mx/vir/structure/structures.html), blast for Ig sequence (http://www.ncbi.nlm.nih.gov/igblast) and the like (Fig. 15).

The positions of the CDRs 1 - 3 of the light chain were determined by reference to the variable region (Rattus norvegicus (hybridoma 53R-4) immunoglobulin rearranged kappa-chain mRNA variable (V) region, partial ds (http://www.ncbi.nlm.nih.gov/nuccore/L07407.1)) (SEQ ID NO: 27) of the conventional antibody. In addition, they were determined in the same manner as in the heavy chain, by using the light chain of C2IgG1 described in patent document 3 (SEQ ID NO: 83 in patent document 3; SEQ ID NO: 28 in the present description), which is the conventional anti-CD98 antibody (Fig. 16).

The amino acid sequences of CDR1 - CDR3 in the heavy chain variable region and light chain variable region of the 3-142 antibody, and CDR1 - CDR3 in the heavy chain variable region and light chain variable region of the hCD98-314 antibody were the sequences shown by the following SEQ ID NOs, respectively.

<Heavy chain variable region CDR1 of 3-142 antibody>
   DYYMA (SEQ ID NO: 13)
<Heavy chain variable region CDR2 of 3-142 antibody>
   SISYEGSSIYYGDSVKG (SEQ ID NO: 14)
<Heavy chain variable region CDR3 of 3-142 antibody>
   RGYYGYKPFDY (SEQ ID NO: 15)
<Light chain variable region CDR1 of 3-142 antibody >
   RASEDIYNGLA (SEQ ID NO: 16)
<Light chain variable region CDR2 of 3-142 antibody>
   NIDNLHT (SEQ ID NO: 17)
<Light chain variable region CDR3 of 3-142 antibody>
   QQYYTYAYT (SEQ ID NO: 18)
<Heavy chain variable region CDR1 of hCD98-314 antibody>
   NYYMA (SEQ ID NO: 19)
<Heavy chain variable region CDR2 of hCD98-314 antibody>
   SISAGGVNNYYRDSMKG (SEQ ID NO: 20)
<Heavy chain variable region CDR3 of hCD98-314 antibody>
   LVNNLGTNVFPD (SEQ ID NO: 21)
<Light chain variable region CDR1 of hCD98-314 antibody>
   KSSQSLLSSGNQKNYLA (SEQ ID NO: 22)
<Light chain variable region CDR2 of hCD98-314 antibody>
   WASTRQS (SEQ ID NO: 23)
<Light chain variable region CDR3 of hCD98-314 antibody>
   QQYKEIPLT (SEQ ID NO: 24)

### [Sequence Listing Free Text]

SEQ ID NO: 9: primer (γ chain)
SEQ ID NO: 10: primer (γ chain)
SEQ ID NO: 11: primer (κ chain)
SEQ ID NO: 12: primer (κ chain)

### Industrial Applicability

In the case of, for example, treatment of diabetes (particularly type 1 diabetes), the anti-CD98 antibody of the present invention and a therapeutic agent for an autoimmune disease using the same have enabled a new antibody therapy of diabetes since they can suppress necrosis or depletion of pancreatic β cell. Moreover, in the case of, for example, treatment of rheumatoid arthritis and the like, a new antibody therapy of rheumatism has been enabled, since the onset and progression of arthritis can be suppressed. Thus, a therapeutic agent for an autoimmune disease, which contains the anti-CD98 antibody of the present invention, is extremely highly useful as a therapeutic drug that does not influence the energy metabolism and causes less side effects for autoimmune diseases for which there is no conventional appropriate treatment method.

## Claims

1. An anti-CD98 antibody or a functional antigen-binding fragment thereof, which comprises the sequence shown by SEQ ID NO: 19 as CDR1 of a heavy chain, the sequence shown by SEQ ID NO: 20 as CDR2 of a heavy chain, and the sequence shown by SEQ ID NO: 21 as CDR3 of a heavy chain, the sequence shown by SEQ ID NO: 22 as CDR1 of a light chain, the sequence shown by SEQ ID NO: 23 as CDR2 of a light chain, and the sequence shown by SEQ ID NO: 24 as CDR3 of a light chain.

2. The anti-CD98 antibody or a functional antigen-binding fragment thereof according to claim 1, which comprises 2 sequences of SEQ ID NOs: 6 and 8 as a heavy chain variable region and a light chain variable region, respectively.

3. The anti-CD98 antibody or a functional antigen-binding fragment thereof according to claim 1 or 2, wherein the anti-CD98 antibody is produced by NITE BP-1007.

4. The anti-CD98 antibody or a functional antigen-binding fragment thereof according to any one of claims 1 to 3, wherein the class of the antibody heavy chain constant region is IgG.

5. The anti-CD98 antibody or a functional antigen-binding fragment thereof according to any one of claims 1 to 4, wherein the aforementioned functional fragment is selected from the group consisting of heavy chain and light chain variable regions (V_{H} and V_{L}), Fab, Fab', F(ab')₂, Fv, scFv, sdFv of the antibody and combinations thereof.

6. An anti-CD98 antibody or a functional antigen-binding fragment thereof, which comprises the sequence shown by SEQ ID NO: 13 as CDR1 of a heavy chain, the sequence shown by SEQ ID NO: 14 as CDR2 of a heavy chain, and the sequence shown by SEQ ID NO: 15 as CDR3 of a heavy chain, the sequence shown by SEQ ID NO: 16 as CDR1 of a light chain, the sequence shown by SEQ ID NO: 17 as CDR2 of a light chain, and the sequence shown by SEQ ID NO: 18 as CDR3 of a light chain.

7. The anti-CD98 antibody or a functional antigen-binding fragment thereof according to claim 6, which comprises 2 sequences of SEQ ID NOs: 2 and 4 as a heavy chain variable region and a light chain variable region, respectively.

8. The anti-CD98 antibody or a functional antigen-binding fragment thereof according to claim 6 or 7, wherein the class of the antibody heavy chain constant region is IgG.

9. The anti-CD98 antibody or a functional antigen-binding fragment thereof according to any one of claims 6 to 8, wherein the aforementioned functional fragment is selected from the group consisting of heavy chain and light chain variable regions (V_{H} and V_{L}), Fab., Fab', F(ab')₂, Fv, scFv, sdFv of the antibody and combinations thereof.

10. The anti-CD98 antibody or a functional antigen-binding fragment thereof according to any one of claims 6 to 9, wherein the anti-CD98 antibody is produced by NITE BP-964.

11. A pharmaceutical composition comprising the anti-CD98 antibody or a functional antigen-binding fragment thereof according to any one of claims 1 to 10 as an active ingredient.

12. The anti-CD98 antibody or a functional antigen-binding fragment thereof according to any one of claims 1 to 10, for use in the prophylaxis or treatment of an autoimmune disease.

13. The anti-CD98 antibody or a functional antigen-binding fragment thereof for use according to claim 12, wherein the autoimmune disease is type 1 diabetes, rheumatoid arthritis, ulcerative colitis, psoriasis, Crohn's disease, systemic lupus erythematosus or Basedow's disease.

## Patentansprüche

1. Anti-CD98-Antikörper oder funktionelles antigenbindendes Fragment davon, umfassend die in SEQ ID Nr. 19 als CDR1 einer schweren Kette gezeigte Sequenz, die in SEQ ID Nr. 20 als CDR2 einer schweren Kette gezeigte Sequenz, die in SEQ ID Nr. 21 als CDR3 einer schweren Kette gezeigte Sequenz, die in SEQ ID Nr. 22 als CDR1 einer leichten Kette gezeigte Sequenz, die in SEQ ID Nr. 23 als CDR2 einer leichten Kette gezeigte Sequenz und die in SEQ ID Nr. 24 als CDR3 einer leichten Kette gezeigte Sequenz.

2. Anti-CD98-Antikörper oder funktionelles antigenbindendes Fragment davon gemäß Anspruch 1, der oder das die 2 Sequenzen SEQ ID Nr. 6 als variablen Bereich der schweren Kette und SEQ ID Nr. 8 als variablen Bereich der leichten Kette umfasst.

3. Anti-CD98-Antikörper oder funktionelles antigenbindendes Fragment davon gemäß Anspruch 1 oder 2, wobei der Anti-CD98-Antikörper von NITE BP-1007 produziert wird.

4. Anti-CD98-Antikörper oder funktionelles antigenbindendes Fragment davon gemäß einem der Ansprüche 1 bis 3, wobei es sich bei der Klasse des konstanten Bereichs der schweren Kette des Antikörpers um IgG handelt.

5. Anti-CD98-Antikörper oder funktionelles antigenbindendes Fragment davon gemäß einem der Ansprüche 1 bis 4, wobei das oben genannte funktionelle Fragment aus der Gruppe ausgewählt ist, die aus variablen Bereichen der schweren Kette und der leichten Kette (V_{H} und V_{L}), Fab, Fab', F(ab')₂, Fv, scFv, sdFv des Antikörpers und Kombinationen davon besteht.

6. Anti-CD98-Antikörper oder funktionelles antigenbindendes Fragment davon, umfassend die in SEQ ID Nr. 13 als CDR1 einer schweren Kette gezeigte Sequenz, die in SEQ ID Nr. 14 als CDR2 einer schweren Kette gezeigte Sequenz, die in SEQ ID Nr. 15 als CDR3 einer schweren Kette gezeigte Sequenz, die in SEQ ID Nr. 16 als CDR1 einer leichten Kette gezeigte Sequenz, die in SEQ ID Nr. 17 als CDR2 einer leichten Kette gezeigte Sequenz und die in SEQ ID Nr. 18 als CDR3 einer leichten Kette gezeigte Sequenz.

7. Anti-CD98-Antikörper oder funktionelles antigenbindendes Fragment davon gemäß Anspruch 6, der oder das die 2 Sequenzen SEQ ID Nr. 2 als variablen Bereich der schweren Kette und SEQ ID Nr. 4 als variablen Bereich der leichten Kette umfasst.

8. Anti-CD98-Antikörper oder funktionelles antigenbindendes Fragment davon gemäß Anspruch 6 oder 7, wobei es sich bei der Klasse des konstanten Bereichs der schweren Kette des Antikörpers um IgG handelt.

9. Anti-CD98-Antikörper oder funktionelles antigenbindendes Fragment davon gemäß einem der Ansprüche 6 bis 8, wobei das oben genannte funktionelle Fragment aus der Gruppe ausgewählt ist, die aus variablen Bereichen der schweren Kette und der leichten Kette (V_{H} und V_{L}), Fab, Fab', F(ab')₂, Fv, scFv, sdFv des Antikörpers und Kombinationen davon besteht.

10. Anti-CD98-Antikörper oder funktionelles antigenbindendes Fragment davon gemäß einem der Ansprüche 6 bis 9, wobei der Anti-CD98-Antikörper von NITE BP-964 produziert wird.

11. Pharmazeutische Zusammensetzung, die den Anti-CD98-Antikörper oder ein funktionelles antigenbindendes Fragment davon gemäß einem der Ansprüche 1 bis 10 als Wirkstoff umfasst.

12. Anti-CD98-Antikörper oder funktionelles antigenbindendes Fragment davon gemäß einem der Ansprüche 1 bis 10 zur Verwendung bei der Prophylaxe oder Behandlung einer Autoimmunkrankheit.

13. Anti-CD98-Antikörper oder funktionelles antigenbindendes Fragment davon zur Verwendung gemäß Anspruch 12, wobei es sich bei der Autoimmunkrankheit um Typ-1-Diabetes, rheumatoide Arthritis, ulzerative Kolitis, Psoriasis, Morbus Crohn, systemischen Lupus erythematodes oder Morbus Basedow handelt.

## Revendications

1. Anticorps anti-CD98 ou un de ses fragments fonctionnels de liaison à l'antigène, qui comprend la séquence indiquée par SÉQ ID NO: 19 en tant que CDR1 de chaîne lourde, la séquence indiquée par SÉQ ID NO: 20 en tant que CDR2 de chaîne lourde et la séquence indiquée par SÉQ ID NO: 21 en tant que CDR3 de chaîne lourde, la séquence indiquée par SÉQ ID NO: 22 en tant que CDR1 de chaîne légère, la séquence indiquée par SÉQ ID NO: 23 en tant que CDR2 de chaîne légère et la séquence indiquée par SÉQ ID NO: 24 en tant que CDR3 de chaîne légère.

2. Anticorps anti-CD98 ou un de ses fragments fonctionnels de liaison à l'antigène selon la revendication 1, qui comprend 2 séquences de SEQ ID NO: 6 et 8 respectivement en tant que région variable de chaîne lourde et région variable de chaîne légère.

3. Anticorps anti-CD98 ou un de ses fragments fonctionnels de liaison à l'antigène selon la revendication 1 ou 2, l'anticorps anti-CD98 étant produit par NITE BP-1007.

4. Anticorps anti-CD98 ou un de ses fragments fonctionnels de liaison à l'antigène selon l'une quelconque des revendications 1 à 3, dans lequel la classe de la région constante de chaîne lourde d'anticorps est IgG.

5. Anticorps anti-CD98 ou un de ses fragments fonctionnels de liaison à l'antigène selon l'une quelconque des revendications 1 à 4, le fragment fonctionnel susmentionné étant choisi dans le groupe constitué des régions variables des chaînes lourdes et des chaînes légères (V_{H} et V_{L}), des fragments Fab, Fab', F(ab')₂, Fv, scFv, sdFv de l'anticorps et des combinaisons de ceux-ci.

6. Anticorps anti-CD98 ou un de ses fragments fonctionnels de liaison à l'antigène, qui comprend la séquence indiquée par SÉQ ID NO: 13 en tant que CDR1 de chaîne lourde, la séquence indiquée par SÉQ ID NO: 14 en tant que CDR2 de chaîne lourde et la séquence indiquée par SÉQ ID NO: 15 en tant que CDR3 de chaîne lourde, la séquence indiquée par SÉQ ID NO: 16 en tant que CDR1 de chaîne légère, la séquence indiquée par SÉQ ID NO: 17 en tant que CDR2 de chaîne légère et la séquence indiquée par SÉQ ID NO: 18 en tant que CDR3 de chaîne légère.

7. Anticorps anti-CD98 ou un de ses fragments fonctionnels de liaison à l'antigène selon la revendication 6, qui comprend 2 séquences de SÉQ ID NO: 2 et 4 respectivement en tant que région variable de chaîne lourde et région variable de chaîne légère.

8. Anticorps anti-CD98 ou un de ses fragments fonctionnels de liaison à l'antigène selon la revendication 6 ou 7, dans lequel la classe de la région constante de chaîne lourde d'anticorps est IgG.

9. Anticorps anti-CD98 ou un de ses fragments fonctionnels de liaison à l'antigène selon l'une quelconque des revendications 6 à 8, le fragment fonctionnel susmentionné étant choisi dans le groupe constitué des régions variables des chaînes lourdes et des chaînes légères (V_{H} et V_{L}), des fragments Fab, Fab', F(ab')₂, Fv, scFv, sdFv de l'anticorps et des combinaisons de ceux-ci.

10. Anticorps anti-CD98 ou un de ses fragments fonctionnels de liaison à l'antigène selon l'une quelconque des revendications 6 à 9, l'anticorps anti-CD98 étant produit par NITE BP-964.

11. Composition pharmaceutique comprenant en tant que principe actif l'anticorps anti-CD98 ou un de ses fragments fonctionnels de liaison à l'antigène selon l'une quelconque des revendications 1 à 10.

12. Anticorps anti-CD98 ou un de ses fragments fonctionnels de liaison à l'antigène selon l'une quelconque des revendications 1 à 10, destiné à être utilisé dans la prophylaxie ou le traitement d'une maladie auto-immune.

13. Anticorps anti-CD98 ou un de ses fragments fonctionnels de liaison à l'antigène destiné à être utilisé selon la revendication 12, la maladie auto-immune étant le diabète de type 1, la polyarthrite rhumatoïde, la colite ulcéreuse, le psoriasis, la maladie de Crohn, le lupus érythémateux systémique ou la maladie de Basedow.
